(19) Europäisches Patentamt
European Patent Office
Office européen des brevets

(11) Veröffentlichungsnummer : **0 057 885**
**B1**

(12) **EUROPÄISCHE PATENTSCHRIFT**

(45) Veröffentlichungstag der Patentschrift : **09.01.85**

(51) Int. Cl.⁴ : **C 07 D498/10**, C 08 K 5/35, C 08 G 63/68, C 08 G 69/02

(21) Anmeldenummer : **82100675.6**

(22) Anmeldetag : **30.01.82**

(54) **Substituierte Diazaspirodecane, ihre Herstellung, ihre Verwendung als Stabilisatoren für organische Polymere, sowie die so stabilisierten Polymere.**

(30) Priorität : **07.02.81 DE 3104294**

(43) Veröffentlichungstag der Anmeldung : **18.08.82 Patentblatt 82/33**

(45) Bekanntmachung des Hinweises auf die Patenterteilung : **09.01.85 Patentblatt 85/02**

(84) Benannte Vertragsstaaten : **AT BE CH DE FR GB IT LI NL SE**

(56) Entgegenhaltungen : **DE-B- 2 264 582**

(73) Patentinhaber : **HOECHST AKTIENGESELLSCHAFT**
**Postfach 80 03 20**
**D-6230 Frankfurt am Main 80 (DE)**

(72) Erfinder : **Wiezer, Hartmut, Dr.**
**Am Stocket 18**
**D-8901 Lützelburg (DE)**
Erfinder : **Pfahler, Gerhard, Dr.**
**Karlsbader Strasse 27**
**D-8900 Augsburg (DE)**
Erfinder : **Korbanka, Helmut, Dr.**
**Birkenstrasse 24**
**D-8901 Adelsried (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 057 885 B1

Jouve, 18, rue St-Denis, 75001 Paris, France

**Beschreibung**

Die vorliegende Erfindung betrifft Verbindungen der Formel (I)

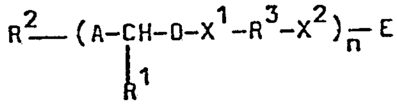

(I)

worin

n eine ganze Zahl von 1 bis 100, vorzugsweise von 1 bis 50 und insbesondere von 1 bis 20 ist,

$X^1$ und $X^2$ entweder gleich sind und für eine Bindung, eine CO-Gruppe oder eine —NHCO-Gruppe mit an den Stickstoff gebunden am $R^3$ stehen, oder, wenn E ohne Bedeutung und n = 1 ist, verschieden sind, wobei $X^2$ dann ohne Bedeutung ist.

$R^1$ steht für Wasserstoff, $C_1$- bis $C_{30}$, vorzugsweise $C_1$- bis $C_{18}$-Alkyl, und insbesondere Methyl, für Phenyl oder insbesondere für eine Gruppe der Formel (IIa) oder vorzugsweise (IIb)

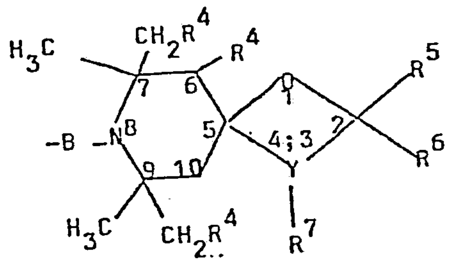

(IIa)

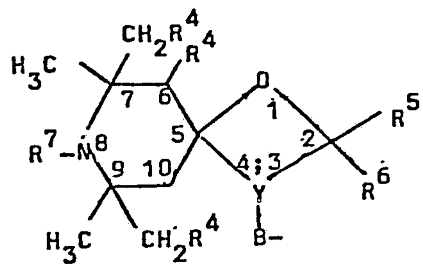

(IIb)

in welcher

Y ein Radikal 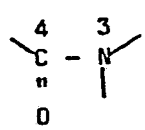 oder 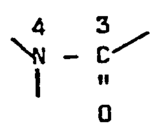

sein soll, das die Ringposition 3 ; 4 ; einnimmt.

B ist eine Bindung oder eine —$CH_2$-Gruppe, vorzugsweise diese und

$R^4$ stellt Wasserstoff oder Methyl, insbesondere Wasserstoff dar.

$R^5$ und $R^6$ sind gleich oder verschieden, und bedeuten Wasserstoff, $C_1$- bis $C_{18}$-, vorzugsweise $C_1$- bis $C_{13}$- und insbesondere $C_1$- bis $C_9$-Alkyl, unsubstituiertes oder durch Chlor oder $C_1$- bis $C_4$-Alkyl substituiertes Phenyl, unsubstituiertes oder durch $C_1$- bis $C_4$-Alkyl substituiertes $C_7$- bis $C_{14}$-Aralkyl, vorzugsweise $C_7$- bis $C_9$-Phenylalkyl, oder auch zusammen mit dem diese Reste bindenden Kohlenstoffatom einen unsubstituierten oder durch bis zu vier $C_1$- bis $C_4$-Alkylgruppen substituierten $C_5$- bis $C_{12}$-Cycloalkyl- oder Piperidinring.

$R^7$ ist Wasserstoff oder $C_1$- bis $C_4$-Alkyl, vorzugsweise Wasserstoff.

Wenn n = 1 ist, steht

$R^2$ für Wasserstoff, für eine Gruppe der Formeln II, für Phenyl, für $C_1$- bis $C_{30}$-, vorzugsweise $C_1$- bis $C_{18}$-Alkyl, für eine $C_1$- bis $C_{18}$-alkylsubstituierte, $C_5$- oder $C_6$-cycloalkylsubstituierte oder phenylsubstituierte Acyl- oder Carbamoylgruppe, deren 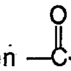-Gruppe an A gebunden ist, wobei

A dann eine Bindung, oder —$CH_2$— oder —$CH_2O$— mit an $R^2$ gebundenem Sauerstoff ist und wenn A und B eine Bindung sind, $R^2$ vorzugsweise Sauerstoff oder Methyl darstellt.

Wenn n = 2 ist, steht

$R^2$ für $C_2$- bis $C_{18}$-, vorzugsweise $C_2$- bis $C_{12}$-Alkylen, für unsubstituiertes oder durch bis zu zwei $C_1$- bis $C_4$- vorzugsweise $C_1$-Alkylgruppen substituiertes Phenylen, für $\alpha,\omega$-Dicarboxi-$C_1$- bis $C_8$-Alkylen, für einen Dicarboxy-$C_6$-Ring, für $C_7$- bis $C_{14}$-Aralkylen, für einen Rest $\geq$N-Alkyl mit 1 bis 6 C-Atomen, oder für eine Gruppe der Formel (III)

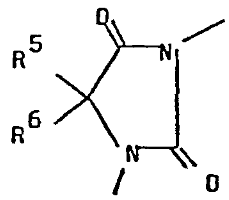

(III)

in welcher $R^5$ und $R^6$ die oben angegebenen Bedeutungen haben, und

A bedeutet dann, $—CH_2—$, oder $—OCH_2—$ mit an $R^2$ gebundenem —O—, und wenn $R^2$ eine Gruppe der Formel (III) ist, steht A für $—CH_2—$. Wenn n = 3 ist, und A sowie B eine $—CH_2$-Gruppe darstellen, bedeutet

$R^2$ einen Isocyanursäurerest, ein Stickstoffatom oder einen

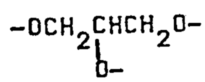 Rest

Wenn $X^2$ und E ohne Bedeutung sind, ist

$R^3$ Wasserstoff, $C_1$- bis $C_{18}$-, vorzugsweise $C_1$- bis $C_{12}$-Alkyl, unsubstituiertes oder durch Chlor, Hydroxi, Amino oder $C_1$- bis $C_4$-Alkyl substituiertes Phenyl, $C_7$- bis $C_{14}$-Aralkyl vorzugsweise Phenylalkyl, oder $C_5$- oder $C_6$-Cycloalkyl, oder steht für $C_2$- bis $C_{12}$-Alkylen, für unsubstituiertes oder für durch eine bis vier $C_1$- bis $C_4$-Alkylgruppen vorzugsweise Methylgruppen substituiertes, Phenylen oder für $C_7$- bis $C_{14}$-Aralkylen vorzugsweise $C_7$ bis $C_9$-Phenylalkylen.

A hat außerdem die Bedeutung einer Gruppe der Formel (IV)

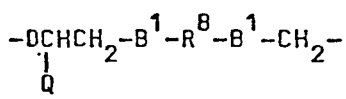

(IV)

worin Q für eine Gruppe der Formeln (II) mit

B = $—CH_2—$ steht,

$B^1$ eine Bindung oder Sauerstoff ist, $R^8$ die Bedeutung eines bi- oder trifunktionellen $R^2$-Restes hat, in welchem bei n = 3 die freie Valenz durch die Gruppe

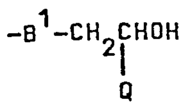

abgesättigt, und $—CH_2—$ an 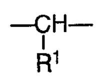 gebunden ist.

A steht außerdem für eine Gruppe der Formel (Va)

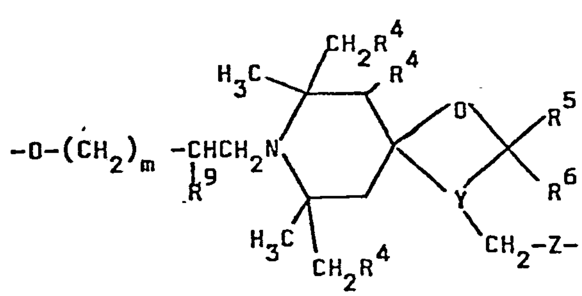

(Va)

in welcher $R^4$, $R^5$, $R^6$ und Y die oben angegebenen Bedeutungen haben, Z an $—CHR^1—$ in Formel (I) gebunden ist und für eine Bindung oder —CH(OH)— steht.

Wenn m = 0 ist, so hat $R^1$ in Formel I die oben angegebenen Bedeutungen, ist $R^9$ gleich $R^1$ und steht Z für eine Bindung.

Ist m = 1, so steht $R^9$ für eine OH-Gruppe, Z für die Gruppe —CH(OH) und $R^1$ in Formel (I) für Wasserstoff. Wenn $R^1$ nicht die Bedeutung einer Gruppe der Formeln (II) hat, steht A auch für die Gruppen der Formeln (Vb) oder (Vc)

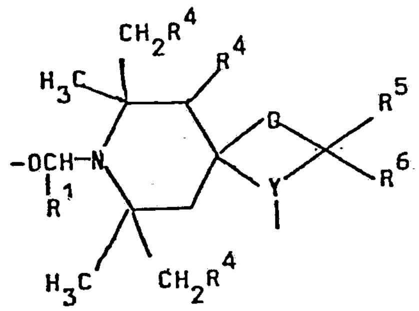

(Vb)

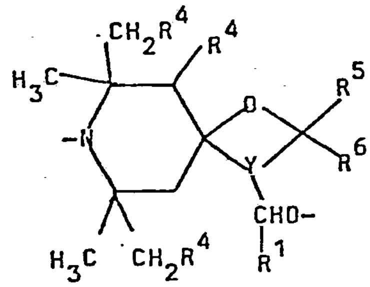

(Vc)

worin $R^1$, $R^4$, $R^5$, $R^6$ und Y die oben angegebenen Bedeutungen, mit Ausnahme von $R^1$ = Verbindung der Formeln (II), haben, $R^1$ vorzugsweise Wasserstoff sein soll und der Sauerstoff an $R^2$ gebunden ist.

In oligomeren oder polymeren Produkten mit $n > 1$ ist

$R^2$ als Endgruppe Wasserstoff,

$R^3$ ein multifunktioneller Rest wie für n = 2, 3 angegeben und hat

A die für n = 2, 3 angegebenen Bedeutungen.

E hat schließlich bei n = 1 die Bedeutung einer Gruppe der Formel

$$R^2—A—CH(R^1)—O—$$

worin $R^1$, $R^2$ und A die oben für n = 1 angegebenen Bedeutungen haben, oder E hat keine Bedeutung, oder E hat als Endgruppe für $n > 1$ die Bedeutung von Niedrigalkylester in Form des Methyl- oder Ethylesters, —NCO oder Halogen, vorzugsweise Cl, Br oder J besitzen, wobei $X^2$ dann ohne Bedeutung ist.

$R^2$ ist als Endgruppe in Polymeren Wasserstoff.

Mindestens einer der Reste $R^1$, $R^2$ oder A muß ein Diazaspirodecansystem der Formeln (II) oder (V) enthalten.

Die neuen Verbindungen werden aus Diazaspirodecanen der allgemeinen Formeln (VI), die in den DE-OSS 26 06 026 und 26 34 957 beschrieben sind

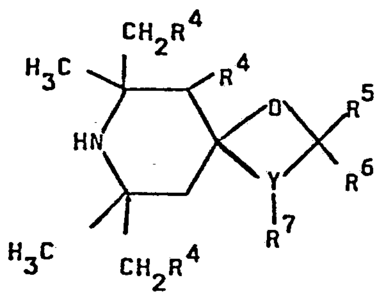

(VIa)

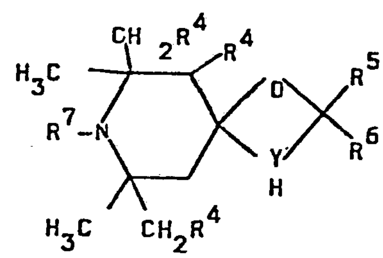

(VIb)

und worin $R^4$, $R^5$, $R^6$, $R^7$ und Y die oben angegebenen Bedeutungen haben, erhalten. Man kann hierbei 2 Wege einschlagen.

Weg a) besteht darin, Verbindungen (VIa) oder (VIb) in Gegenwart von Katalysatoren mit der 1,0 bis 1,5 fach, vorzugsweise 1,0 bis 1,2 fach und insbesondere äquivalente Menge eines Epoxids der Formel (VIIa)

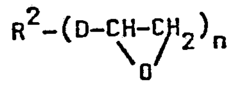

(VIIa)

oder der 1,0 bis 3 fach, vorzugsweise 1,0 bis 1,5 fach und insbesondere äquivalenten Menge eines Aldehyds der Formel (VIIb),

$$R^2—(CHO)_n \quad (VIIb)$$

umzusetzen, wobei $R^2$ in den Formeln (VII) die oben angegebene Bedeutung hat, n eine ganze Zahl von 1 bis 3 ist und D für eine Bindung oder Methylen oder $—OCH_2—$ mit an $R^2$ gebundenem —O— steht. In

Formel (VIIa) hat $R^2$ zusätzlich die Bedeutung einer Gruppe der Formeln (II) mit B = Bindung, wobei dann in (VIIa) n = 1 und D = $CH_2$ ist, worauf man die so erhaltenen Alkohole gewünschtenfalls zur Synthese von Folgeprodukten definierten Molgewichts mit der äquivalenten, bei Polyolen zur Synthese oligomerer oder polymerer Folgeprodukte mit der äquimolaren Menge einer bifunktionellen Verbindung der Formel (VIII)

$$R^3—(T)_m \quad (VIII)$$

in welcher $R^3$ die oben angegebene Bedeutung hat, m eine ganze Zahl von 1 bis 3 ist und T für Halogen, Niedrigalkylester, —COCl, OH oder —NCO steht, umsetzt.

Bei Weg b) geht man von Epoxiverbindungen der Formel (VIIa), worin $R^2$ eine Gruppe der Formeln (IIa), oder (IIb) ist, in der B = eine Bindung, D = —$CH_2$— und n = 1 ist, aus, (s. DE-OS 28 34 962) und setzt diese mit Ameisensäure die in der 3 bis 10 fach molaren Menge anwesend sein soll und gleichzeitig als Solvens dient, bei 100 °C um. Die hierbei resultierenden Ameisensäureester werden anschließend nach bekannten Methoden alkalisch zum Alkohol verseift, und, falls gewünscht, wie bei Methode a) beschrieben zu Folgeprodukten weiterverarbeitet.

Soll der Reaktionsweg a) beschritten werden, so arbeitet man, wenn eine Verbindung der Formel (VIIa) eingesetzt wird, in inerten organischen Lösungsmitteln wie z. B. aliphatischen Alkoholen in Gegenwart von 0,1 bis 2 Gew.-% eines basischen Katalysators, wobei vorzugsweise 0,1 bis 3 % eines Phasentransferkatalysators zugegen sind, wenn die Reaktion an $\gtrdot$Y—H, stattfinden soll, und mit sauren Katalysatoren, bei gewünschter Umsetzung am basischen $\gtrdot$NH. Ist eine Verbindung der Formel (VIIb) umzusetzen, so verwendet man generell basische Katalysatoren in der oben angegebenen Menge und kann dann auch in Wasser arbeiten. Die Reaktionstemperaturen liegen zwischen 50 und 180, vorzugsweise 70 und 160 °C.

Die Umsetzung der in dieser Stufe gebildeten Alkohole zu Folgeprodukten wird bei Temperaturen von 80 bis 180, vorzugsweise 100 bis 160 °C in inerten organischen Lösungsmitteln wie z. B. aromatischen Kohlenwasserstoffen vorgenommen, bei T = Halogen unter vorheriger Bildung der Alkalialkoholate der eingesetzen Alkohole, bei T = Niedrigalkylester unter Verwendung von 0,1 bis 0,5 Gew.-% eines basischen Katalysators wie z. B. $LiNH_2$, NaH, $NaOCH_3$ oder $KOCH_3$, bei T = —NCO unter Verwendung von 0,1 bis 0,5 Gew.-% eines basischen Katalysators wie KOH oder 1,4-Diazabicyclo-[2.2.2]-octan und bei T = —COCl unter Verwendung der äquivalenten Menge eines Halogenwasserstoff-Akzeptors. Wenn aus (VIIb) erhaltene Alkohole veräthert werden, arbeitet man in Gegenwart von sauren Katalysatoren, vorzugsweise mit Chlorwasserstoff bei einem pH von ca. 2.

Zu Reaktionsweg b), der in seinem 2. Teil dem des 2. Teils des Reaktionsweges a) entspricht, sind keine zusätzlichen Ausführungen erforderlich.

Die aus den DE-OSS 26 06 026 und 26 34 957 bekannten Verbindungen, die in vorliegenden Falle Ausgangsmaterialien der Formel (VI) sind, und die ansich bereits gute Stabilisatoren darstellen, befriedigen besonders was die Verträglichkeit mit den zu stabilisierenden Polymeren und die Flüchtigkeit betrifft, nicht in vollem Maße. Konstitutionell ferner stehende Piperidinstabilisatoren sind aus der DE-B-2 264 582 bekannt, welche von Dioxo-spiro-decanen abgeleitet sind.

Aus der EP-Patentanmeldung 17 617 sind ebenfalls Verbindungen bekannt, die aus den vorstehend genannten Ausgangsmaterialien zugänglich sind. Diese Verbindungen sind unzureichend wirksam, weil das für die Wirksamkeit als Stabilisator verantwortliche Zentrum bei den interessanten, hochmolekularen Produkten mit $n > 1$ blockiert ist.

Stabilisatoren, die andere Piperidinverbindungen als Bausteine enthalten und oligomer aufgebaute Substanzen darstellen, sind aus der DE-OS 27 19 131 und der EP-Anmeldung 2005 bekannt. Diese Produkte besitzen keine ausreichende Lichtschutzwirkung. Demgegenüber übertreffen die neuen Verbindungen die aus vorgenannten Anmeldungen bekannten Stabilisatoren in allen ihren Eigenschaften, was nicht erwartet werden konnte. Desweiteren war nicht zu erwarten, daß die Reaktion a) der Produktherstellung so einfach durch geeignete Katalysatorwahl zu steuern war.

Beispiele für die als Ausgangsprodukte dienenden Verbindungen der Formeln (VI) sind die in den DE-OSS 26 06 026, 26 34 957 und 28 34 962 beschriebenen Diazaspirodecane.

Beispiele für Epoxide der Formel (VIIa) sind :

Ethylenoxid, Propylenoxid, Glycid, Tris-(2,3-epoxipropyl)-isocyanurat, 1,4-Bis-(2,3-epoxipropyloxi)-butan, Cyclohexan-1,2-di-(2,3-epoxipropyl)-carbonsäureester, Tetrahydrophthalsäuredi-(2,3-epoxipropyl)-ester, Bis-(2,3-epoxipropyl)-aminobutan, Tris-(2,3-epoxipropyl)-amin, 2,3-Bis-[4-(2,3-epoxipropyl)-phenyl]-propan, 5,5-Dimethyl-1,3-[bis-(2,3-epoxipropyl)]-diazapentandion-2,4, Epoxide wie sie in DE-OS 29 41 004 beschrieben sind, 1,2,3,4-Di-epoxibutan.

Aldehyde der Formel (VIIb) haben 1 bis 12 C-Atome. Genannt seien bevorzugt Formaldehyd und Acetaldehyd.

Beispiele für Verbindungen der Formel (VIII) sind : Brombutan, Dibromhexan, Dibromdecan, Jodmethan, Essigsäure-ethylester, Oenanthsäuremethylester, Laurinsäuremethylester, Benzoesäuremethylester, Malonsäurediethylester, Bernsteinsäurediethylester, Adipinsäurediethylester, Sebazinsäuredimethylester, Terephthalsäuredimethylester, Benzoltricarbonsäuretrimethylester, Adipinsäuredichlorid,

Sebazinsäuredichlorid, Cyclohexylisocyanat, Butylisocyanat, Octadecylisocyanat, Hexamethylendiisocyanat, p-Tolylisocyanat, Phenylisocyanat und Tolylen-2,4-diisocyanat.

Die neuen Stabilisatoren lassen sich problemlos in die zu stabilisierenden Polymeren einarbeiten und sind hervorragend zum Stabilisieren derselben gegen den lichtinduzierten oxidativen Abbau d. h. ihre Schädigung durch Einwirkung von Sauerstoff, Wärme und Licht geeignet. Neben der ausgezeichneten Stabilisatorwirksamkeit zeichnet die neuen Stabilisatoren ihre sehr gute Verträglichkeit mit den zu stabilisierenden Polymeren aus.

Beispiele für erfolgreich zu stabilisierende Polymere sind :

Polymere, die sich von einfach oder doppelt ungesättigten Kohlenwasserstoffen ableiten, z. B. Polyolefine wie Polyethylen, das gegebenenfalls vernetzt sein kann, Polypropylen, Polybuten-1, Polyisobuten, Polymethylbuten-1, Polymethylpenten-1, Polyisopren, Polybutadien, Polystyrol, Copolymere der den genannten Homopolymeren zugrundeliegenden Monomeren, wie Ethylen-Propylen-Copolymere, Propylen-Buten-1-Copolymere, Propylen-Isobuten-Copolymere, Styrol-Butadien-Copolymere sowie Terpolymere von Ethylen und Propylen mit einem Dien, wie z. B. Hexadien, Dicyclopentadien oder Ethylidennorbornen ; Mischungen der obengenannten Homopolymeren, wie beispielsweise Gemische von Polypropylen und Polyethylen, Polypropylen und Polybuten-1, Polypropylen und Polyisobutylen oder von Butadien-Acrylnitril-Copolymerisaten mit einem Styrol-Butadien-Copolymerisat.

Halogenhaltige Vinylpolymere, wie Polyvinylchlorid, Polyvinylidenchlorid, Polyvinylfluorid, Polychloropren, Chlorkautschuke sowie Copolymere von Vinylchlorid und Vinylidenchlorid untereinander und mit anderer olefinisch ungesättigten Monomeren.

Polymere, die sich von α,β-ungesättigten Säuren und deren Derivaten ableiten, wie Polyacrylate und Polymethacrylate, Polyacrylamide und Polyacrylnitril sowie deren Copolymere untereinander und mit anderen Vinylverbindungen, wie Acrylnitril-Butadien-Styrol-, Acrylnitril-Styrol- und Acrylnitril-Styrol-Acrylester-Copolymerisate.

Polymere, die sich von ungesättigten Alkoholen und Aminen bzw. deren Acylderivaten oder Acetalen albeiten, wie Polyvinylalkohol, Polyvinylacetat, -stearat, -benzoat, -maleat, Polyvinylbutyral, Polyallylphthalat, Polyallylmelamin und deren Copolymere mit anderen Vinylverbindungen, wie Ethylen-Vinylacetat-Copolymere. Homo- und Copolymere, die sich von Epoxiden ableiten, wie Polyethylenoxid oder die Polymerisate, die sich von Bisglycidylethern ableiten.

Polyacetale, wie Polyoximethylen und Polyoxiethylen sowie solche Polyoximethylene, die als Comonomeres Ethylenoxid enthalten.

Polyurethane und Polyharnstoffe

Polycarbonat

Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12.

Polyester, die sich von Dicarbonsäuren und Dialkoholen und/oder von Hydroxicarbonsäuren oder den entsprechenden Lactonen ableiten, wie Polyethylenterephthalat, Polybutylenterephthalat, Poly-1,4-dimethylol-Cyclohexanterephthalat.

Vernetzte Polymerisate, die sich von Aldehyden einerseits und Phenolen, Harnstoffen und Melaminen andererseits ableiten, wie Phenol-Formaldehyd-, Harnstoff-Formaldehyd- und Melamin-Formaldehydharze.

Die neuen Verbindungen lassen sich schließlich auch als Stabilisatoren auf dem Harz- und Lacksektor einsetzen. Beispiele sind duroplastische und thermoplastische Acrylharze, welche für Autolackierungen verwendet werden (Encyclopedia of Polymer Science and Technology, Interscience Publishers, New York, Band 1 (1964), Seiten 273-276, und Band 13 (1970), Seiten 530-532 ; « Understanding Paint » von W. R. Fuller, in American Paint Journal, St. Louis, 1965, Seiten 124-135), Acrylharzlacke, das sind die üblichen Einbrennlacke (beschrieben z. B. in H. Kittel's « Lehrbuch der Lacke und Beschichtungen », Band 1, Teil 2, Seite 735 und 742 (Berlin, 1972) und in H. Wagner, H. F. Sarx (« Lackkunstharze », Seiten 229-235) sowie ganz besonders Mischungen auf der Basis von heißvernetzbarem Acrylharz und Styrol sowie Lacke und Beschichtungen auf der Basis von Acryl-Melaminharz und Alkyl/Acryl/Melaminharz. Derartige Lacke können als weitere Zusatzstoffe andere übliche Lichtschutzmittel, phenolische Antioxidantien, Pigmente, Farbstoffe, Metalldesaktivatoren etc. enthalten.

Von besonderer Bedeutung ist die Stabilisierung von Polyolefinen, Styrolpolymerisaten, Polyamiden, Poly-(meth)acrylaten und von Polyurethanen, für die sich die Verbindungen bevorzugt eignen. Beispiele hierfür sind Polyethylen hoher und niedriger Dichte, Polypropylen, Ethylen-Propylen-Copolymerisate, Polystyrol, Styrol-Butadien-Acrylnitril-Terpolymerisate ; Mischungen von Polyolefinen oder von Styrolpolymerisaten sowie Polyurethane auf Polyether- oder Polyesterbasis.

Die neuen Stabilisatoren werden nach allgemein üblichen Methoden in die Polymermassen eingearbeitet. Die Einarbeitung kann beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das

Polymere direkt oder Einmischen in eine Lösung, Suspension oder Emulsion desselben, gegebenenfalls unter nachträglichem Verdunstenlassen des Lösungsmittels, erfolgen. Die Mengen liegen bei 0,01 bis 5, vorzugsweise bei 0,05 bis 2,5 und insbesondere bei 0,1 bis 1,0 Gew.-%, bezogen auf das zu stabilisierende Material. Die neuen Verbindungen können auch in Form eines Masterbatches, der diese Verbindungen beispielsweise in einer Konzentration von 1 bis 50, vorzugsweise 2,5 bis 20 Gew.-% enthält, den zu stabilisierenden Kunststoffen zugesetzt werden.

Die durch den Zusatz der erfindungsgemäßen Substanzen stabilisierten Kunststoffe können gegebenenfalls noch andere bekannte und übliche Zusätze, wie beispielsweise Antioxidantien auf Phenol- und Sulfidbasis, Metalldesaktivatoren und Lichtschutzmittel, Phophitstabilisatoren, Metallverbindungen, Epoxistabilisatoren und mehrwertige Alkohole enthalten (s. a. DE-OS 24 27 853, S. 18-24).

Beispiele für Antioxidantien sind sterisch gehinderte Phenole wie 2,6-Di-tert.-butyl-4-methylphenol, 4,4'-Butyliden-bis-(2,6-di-tert.-butylphenol), 4,4'-Thio-bis-(2-tert.-butyl-5-methylphenol), 2,5-Di-tert.-butyl-4-hydroxianisol, 2,2-Bis-(3,5-di-tert.-butyl-2-hydroxibenzyl)-malonsäuredioctadecylester, 1,3,5-Tris-(3,5-di-tert.-butyl-4-hydroxibenzyl)-2,4,6-trimethylbenzol, 2,4,6-Tri-(3,5-di-tert.-butyl-4-hydroxibenzyl)-phenol, phenolische Triazinverbindung wie 1,3,5-Tris-(3,5-di-tert.-butyl-4-hydroxibenzyl)-isocyanurat, Ester der β-(3,5-Di-tert.-butyl-4-hydroxiphenyl)-propionsäure mit z. B. Octadecanol, Pentaerythrit und Tris-hydroxiethyl-isocyanurat, Ester der 3,3-Bis-(3-tert.-butyl-4-hydroxiphenyl)-butansäuren mit z. B. Ethylenglykol, Thiodipropionsäureester mit Fettalkoholen, Ca- oder Ni-Salze des 3,5-Di-tert.-butyl-4-hydroxibenzylphosphonsäureethylesters, Dioctadecylsulfid und -disulfid.

Zu den UV-Absorbern und Lichtschutzmitteln gehören 2-(2'-Hydroxiphenyl)-benztriazole wie z. B. das 5-Chlor-3'-, 5'-di-tert.-butyl- und 5-Chlor-3',5'-di-tert.-amyl-Derivat, 2-Hydroxibenzophenone wie z. B. das 4-Heptoxi- oder 4-Octoxi-Derivat, Salizylate wie Octylphenylsalizylat, Nickelkomplexe wie z. B. der Komplex mit 2,2'-Thio-bis-4-(1,1,3,3-tetramethylbutyl)-phenol und Butylamin oder anderen Aminen, Oxalsäurediamide und sterisch gehinderte Amine.

Als Phosphite sind aliphatische, aromatische oder aliphatisch-aromatische wie z. B. Trisnonylphenylphosphit, Tris-(2,4-di-tert.-butylphenyl)-phosphit, Tris-(2-tert.-butylphenyl)-phosphit oder auch Ester des Pentaerythritphosphits zu nennen.

Als Stabilisatoren bekannte Metallverbindungen sind z. B.: Calcium-, Barium-, Strontium-, Zink-, Cadmium, Magnesium-, Aluminium- und Bleiseifen aliphatischer Carbonsäuren oder Oxicarbonsäuren mit etwa 12 bis 32 C-Atomen, Salze der genannten Metalle mit aromatischen Carbonsäuren wie Benzoate oder Salizylate sowie (Alkyl-)-Phenolate dieser Metalle, ferner Organozinnverbindungen, wie z. B. Dialkylzinnthioglykolate und -carboxilate.

Bekannte Epoxistabilisatoren sind z. B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d. h. bevorzugt Alkohole mit 5 oder 6 C-Atomen und 2 bis 6 OH-Gruppen.

Eine wirksame Stabilisatorkombination für Poly-α-Olefine, wie z. B. Hoch-, Mittel- und Niederdruckpolymerisate von $C_2$- bis $C_4$-α-Olefinen, insbesondere Polyethylen und Polypropylen oder von Copolymerisaten derartiger α-Olefine besteht, bezogen auf 100 Gew.-Teile Polymer, beispielsweise aus 0,01 bis 5 Gewichtsteilen einer der erfindungsgemäß zu verwendenden Verbindungen, 0,05 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z. B. Calciumstearat oder Zinkstearat oder der entsprechenden Oxide sowie gegebenenfalls 0,01 bis 5 Gewichtsteilen eines bekannten UV-Stabilisators aus der Gruppe der Alkoxihydroxibenzophenone, 4-Hydroxiphenylbenzotriazole, Benzylidenmalonsäuremononitrilester oder der sog. Quencher, wie z. B. Nickelchelate. Als sonstige übliche Zusätze sind z. B. Weichmacher, Gleitmittel, Emulgatoren, Füllstoffe wie z. B. Kreide, Talkum, Asbest, Pigmente, optische Aufheller, Flammschutzmittel und Antistatika anzusehen.

Die erfindungsgemäß stabilisierten Kunststoffe können in verschiedenster Form angewendet werden, z. B. als Folien, Fasern, Bändchen, Profile oder als Bindemittel für Lacke Klebemittel oder Kitte.

Der weiteren Erläuterung der Erfindung dienen nachstehende Beispiele.

I. Darstellung von Alkoholen nach Verfahrensweg a)

## Beispiel 1

2,7,7,9,9-Pentamethyl-2-isopropyl-8-(2,3-dihydroxipropyl)-1-oxa-4,8-diaza-spiro-[4.5]-decanon-3.

In einer Rührapparatur werden 26,8 g (0,1 Mol) 2,7,7,9,9-Penta-methyl-2-isopropyl-1-oxa-4,8-diaza-spiro-[4.5]-decanon-3 und 7,4 g (0,1 Mol) Glycid in 60 ml Hexanol unter Verwendung von 3 Tropfen konz. HCl als Katalysator 65 Std. bei 140° gerührt. Anschließend wird einrotiert und der Rückstand aus Xylol/Methanol umkristallisiert.

Ausbeute: 25 g; Fp 183 °C

Beispiele 2 bis 5

Wie in Beispiel 1 angegeben, wurden aus Glycid und anderen Diaza-spiro-decanonen hergestellt :

Beispiel 2

2,7,7,9,9-Pentamethyl-2-hexyl-8-(2,3-dihydroxipropyl)-1-oxa-3,8-diaza-spiro-[4.5]-decanon-4 ; Fp 222 °C.

Beispiel 3

2,2,7,7,9,9-Hexamethyl-8-(2,3-dihydroxipropyl)-1-oxa-3,8-diaza-spiro-[4.5]-decanon-4 ; Fp 256 °C.

Beispiel 4

2,2,4,4-Tetramethyl-3-(2,3-dihydroxipropyl)-7-oxa-3,14-dioxadispiro-[5.1.5.2]-pentadecanon-15 ; Fp 257 °C.

Beispiel 5

2,2,4,4-Tetramethyl-3-(2,3-dihydroxipropyl)-7-oxa-3,20-diaza-di-spiro-[5.1.11.2]-heneicosanon-21 ; Fp 208-215 °C.

Beispiel 6

7,7,9,9-Tetramethyl-2,2-dibenzyl-3-(2,3-dihydroxipropyl)-1-oxa-3,8-diaza-spiro-[4.5]-decanon-4.

39,2 g (0,1 Mol) 7,7,9,9-Pentamethyl-2,2-dibenzyl-1-oxa-3,8-diaza-spiro-[4.5]-decanon-4 werden in 150 ml n-Propanol vorgelegt. Nach Zugabe von 7,8 g (0,105 Mol) Glycid und 1 g Tricaprylmethylammoniumchlorid sowie 1 g 50 %-ige Natronlauge als Katalysatoren wird 16 Std. bei 80 °C gerührt, zur Trockne im Vakuum eingedampft, der Rückstand nach dem Pulverisieren mit Wasser gewaschen und getrocknet.
Ausbeute : 47 g ; Fp 145 °C.

Beispiele 7 bis 9

Analog Beispiel 6 wurden aus Glycid und anderen Diazaspirodecanonen hergestellt :

Beispiel 7

2,2,4,4-Tetramethyl-20-(2,3-dihydroxipropyl)-7-oxa-3,20-diaza-dispiro-[5.1.11.2]-heneicosanon-21 ; Fp 124-131 °C.

Beispiel 8

7,7,9,9-Tetramethyl-2,2-diheptyl-3-(2,3-dihydroxipropyl)-1-oxa-3,8-diaza-dispiro-[4.5]-decanon-4 ; Fp 95-99 °C.

Beispiel 9

7,7,9,9-Tetramethyl-2-iso-nonyl-3-(2,3-dihydroxipropyl)-1-oxa-3,8-diaza-spiro-[4.5]-decanon-4 ; Harz.

Beispiel 10

1,3,5-Tris-[2-hydroxi-3-(2,2,4,4-tetramethyl-7-oxa-3,20-diaza-21-oxo-dispiro-[5.1.11.2]-heneicosyl-20)-propyl]-isocyanurat.

36,4 g (0,1 Mol) 2,2,4,4-Tetramethyl-7-oxa-3,20-diaza-21-oxo-dispiro-[5.1.11.2]-heneicosan, 9,9 g (1/30 Mol) Tris-[2,3-epoxipropyl]-isocyanurat, 1 g Tricaprylmethylammoniumchlorid und 1 g 50 %-ige NaOH werden 30 Stunden in 100 ml Hexanol bei 140 °C gerührt. Nach dem Abkühlen wird filtriert, das Filtrat einrotiert, der Rückstand nach dem Verrühren mit 200 ml Heptan abgenutscht und der Feststoff getrocknet.
Ausbeute : 35 g ; Fp 155-162 °C.

Beispiele 11 bis 15

Es wurden nach der in Beispiel 10 beschriebenen Methode hergestellt :

Beispiel 11

1,4-Bis-[2-hydroxi-3-(2,2,4,4-tetramethyl-7-oxa-3,20-diaza-21-oxo-dispiro-[5,1,11,2]-heneicosy-20)-propyl-oxi]-butan,

ausgehend von dem in Beispiel 10 eingesetzten Heneicosan und 1,4-Bis-(2,3-epoxipropyloxi)-butan. Ausbeute : 51 g ; Fp 85-92 °C.

Beispiel 12

1,2,3-Tris-[2-hydroxi-3-(2,2,4,4-tetramethyl-7-oxa-3,20-diaza-21-oxo-dispiro-[5.1.11.2]-heneicosyl-20)-propyloxi]-propan,

aus Heneicosan und 1,2,3-Tris-(2,3-epoxi-propyloxi)-propan
Ausbeute : 33 g ; Harz.

Beispiel 13

1,2,3-Tris-[2-hydroxi-3-(2,2,7,7,9,9-hexamethyl-1-oxa-4,8-diaza-3-oxo-spiro-[4.5]-decyl-4)-propyloxi]-propan

ausgehend von 2,2,7,7,9,9-Hexamethyl-1-oxa-4,8-diaza-spiro-[4.5]-decanon-3 und 1,2,3-Tris-(2,3-epoxipropyloxi)-propan in n-Propanol als Lösungsmittel bei 80 °C.
Ausbeute : 28 g ; Harz.

Beispiel 14

1,4-Bis-[2-hydroxi-3-(2,7,7,9,9-pentamethyl-2-benzyl-1-oxa-3,8-diaza-4-oxo-spiro-[4.5]-decyl-3-)-propyloxi]-butan,
ausgehend von 2,7,7,9,9-Pentamethyl-2-benzyl-1-oxa-3,8-diaza-spiro-[4.5]-decanon-4 und 1,4-Bis-(2,3-epoxipropyloxi)-butan, analog Bsp. 13.
Ausbeute : 39 g ; Fp 62-65 °C.

Beispiel 15

Cyclohexan-1,2-bis-[2-hydroxi-3-(2,2,4,4,10,10,12-heptamethyl-7-oxa-3-14-diaza-15-oxo-dispiro-[5.1.5.2]-pentadecyl-14)-propyl]-dicarbonsäureester,

ausgehend von 2,2,4,4,10,10,12-Heptamethyl-7-oxa-3,14-diaza-dispiro-[5.1.5.2]-pentadecanon-15 und Cyclohexan 1,2-bis-(2,3-epoxipropyl)-dicarbonsäureester analog Bsp. 13.
Ausbeute : 44 g ; Fp 130 °C.

Beispiel 16

Bis-1,3-[2,2,4,4-tetramethyl-7-oxa-3,20-diaza-21-oxo-dispiro-[5.1.11.2] heneicosyl-2-hydroxi]-propan,

36,4 g (0,1 Mol) des in Bsp. 10 verwendeten Heneicosans werden in 300 ml n-Propanol mit 43,8 g 2,2,4,4-Tetramethyl-20-(2.3-epoxipropyl)-7-oxa-3.20-diaza-dispiro-[5.1.11.2]-heneicosanon-21 unter Zusatz der in Bsp. 10 angegebenen Katalysatoren in den gleichen Mengen 16 Stunden bei 80 °C gerührt. Es wird heiß filtriert, einrotiert, der Rückstand mit Wasser gewaschen und getrocknet.
Ausbeute : 78 g eines farblosen Harzes ; Fp 170 °C, MG 784 Th., 775 gef.

Beispiel 17

7,7,9,9-Tetramethyl-2,2-diethyl-3,8-bis-(2,3-dihydroxipropyl)-1-oxa-3,8-diaza-spiro-[4.5]-decanon-4.

In 60 ml Propanol werden 13,4 g (1/20 Mol) 7,7,9,9-Tetramethyl-2,2-diethyl-1-oxa-3,8-diaza-spiro-[4.5]-decanon-4, 3,7 g (1/20 Mol) Glycid und 1 Tropfen konz. HCl 10 Stunden bei 80 °C gerührt. Anschließend werden weitere 3,7 g Glycid, 0,2 g Tricaprylmethylammoniumchlorid und 0,3 g 50 %-ige NaOH zugegeben, worauf man weitere 15 Stunden bei 80 °C rührt. Anschließend wird filtriert und zur Trockne eingedampft.
Ausbeute : 20,7 g eines Harzes.

II. Darstellung von Alkoholen nach Verfahrensweg b)

Beispiel 18

2,7,7,9,9-Pentamethyl-2-ethyl-3-(2,3-dihydroxipropyl)-1-oxa-3,8-diaza-spiro-[4.5]-decanon-4.

In 200 g Ameisensäure werden 50 g 2,7,7,9,9-Pentamethyl-2-ethyl-3-(2,3-epoxipropyl)-1-oxa-3,8-diaza-spiro-[4,5]-decanon-4 4 Std. am Rückfluß gekocht. Die Ameisensäure wird i. Vak. abdestilliert und

der Rückstand in 100 ml 10 %-iger NaOH 2 Stunden gekocht. Es bildet sich eine ölige Schicht, die abgetrennt wird und nach einiger Zeit erstarrt. Das Produkt wird pulverisiert, mit Wasser gewaschen und getrocknet.

Ausbeute : 45 g ; Fp 121 °C.

Beispiele 19 und 20

Man arbeitet, wie im Beispiel 18 angegeben und erhält aus den entsprechenen 2,3-Epoxipropylverbindungen :

Beispiel 19

7,7,9,9-Tetramethyl-2,2-diethyl-3-(2,3-dihydroxipropyl)-1-oxa-3,8-diaza-spiro-[4.5]-decanon-4 ; Fp 104 °C.

Beispiel 20

2,2,4,4-Tetramethyl-3-(2,3-dihydroxipropyl)-7-oxa-3,20-diaza-di-spiro-[5.1.11.2]-heneicosanon-21 ; Fp 126-130 °C.

III. Herstellung von Folgeprodukten der nach Verfahrensweg a) oder b) erhaltenen Alkohole

Beispiel 21

Bernsteinsäuredi-[bis-1,3-(2,2,4,4-tetramethyl-7-oxa-3,20-diaza-21-oxo-dispiro-[5.1.11.2]-heneicosyl)-2-propyl]-ester.

15,7 g (0,02 Mol) der Verbindung gemäß Bsp. 16, 1,8 g (0,01 Mol) Bernsteinsäurediethylester und 0,1 g $LiNH_2$ werden in 100 ml abs. Mesitylen auf 160 °C erhitzt, wobei im Verlaufe von 3 Std. ca. 0,9 g Ethanol abdestillieren. Anschließend wird zur Trockne eingeengt und der Rückstand mit Wasser gewaschen.

Ausbeute : 15,7 g ; Fp 129 °C.

Beispiel 22

Es wird wie in Beispiel 21 gearbeitet, jedoch unter Einsatz von 0,01 Mol Sebazinsäurediethylester.

Ausbeute : 15,0 g ; Fp 95 °C.

Beispiele 23 bis 31

Nach der Arbeitsweise des Beispiels 21 wurden folgende Polyester hergestellt :

Beispiel 23

Aus Verbindung gemäß Bsp. 1 und Sebazinsäuredimethylester (je 0,015 Mol).
5,7 g eines festen Harzes ; Fp ca 70 °C.

Beispiel 24

Aus Verbindung gemäß Bsp. 2 und Malonsäurediethylester (je 0,02 Mol).
8,0 g eines festen Harzes ; Fp 120-137°C.

Beispiel 25

Aus Verbindung gemäß Bsp. 4 und Adipinsäuredimethylester (je 0,02 Mol).
7,6 g eines festen Harzes ; Fp 94-122 °C.

Beispiel 26

Aus Verbindung gemäß Bsp. 10 und Korksäuredimethylester (je 0,01 Mol).
11,3 g eines festen Harzes ; Fp 125-139 °C.

Beispiel 27

Aus Verbindung gemäß Bsp. 11 und Malonsäurediethylester (je 0,01 Mol).
9,0 g eines festen Harzes ; Fp 118-142 °C.

Beispiel 28

Aus Verbindung gemäß Bsp. 5 und Bernsteinsäurediethylester (je 0,01 Mol) 5,1 g eines festen Harzes ; Fp 123-142 °C.

Beispiel 29

Aus Verbindung gemäß Bsp. 7 und Malonsäurediethylester (je 0,01 Mol). 6 g ; Fp 60-78 °C.

Beispiel 30

Aus Verbindung gemäß Bsp. 7 und Adipinsäuredimethylester (je 0,01 Mol). 6 g ; Fp 51-89 °C.

Beispiel 31

Aus Verbindung gemäß Bsp. 17 und Malonsäurediethylester (je 0,01 Mol). 4 g ; klebriges Harz.

Beispiel 32

Aus der Verbindung nach Bsp. 10 und Cyclohexylisocyanat wurde ein Urethan in folgender Weise erhalten :

In 100 ml Toluol werden 13,9 g (0,01 Mol) der Verbindung nach Bsp. 10 und 3,8 g (1/30 Mol) Cyclohexylisocyanat in Gegenwart von 0,1 g 1,4-Diazinbicyclo-[2.2.2]-octan 15 Std. bei 130 °C gerührt. Sodann wird zur Trockne eingedampft.

17,3 g ; Fp 110 °C.

Beispiel 33

Aus der Verbindung nach Bsp. 11 und Hexamethylendiisocyanat (je 0,02 Mol) erhält man analog Bsp. 32

21,4 g eines Polyurethans vom ; Fp 120-136 °C.

Beispiel 34

Dieses Beispiel zeigt die Flüchtigkeit der neuen Triazinstabilisatoren im Vergleich zu einem Produkt des nächsten Standes der Technik.

Die Flüchtigkeiten wurden in einer Apparatur zur thermogravimetrischen Analyse bestimmt. Gleiche Menge (500 mg) der erfindungsgemäßen Verbindungen und der Vergleichssubstanz werden dazu in Stickstoffatmosphäre mit einer Aufheizgeschwindigkeit von 2 K/min bis auf 300 °C erhitzt und der Substanzverlust in $mg/cm^2$ Probenoberfläche gemessen. Die Ergebnisse zeigt nachstehende Tabelle.

| Stabilisator | Gewichtsverlust in $mg/cm^2$ beim Erreichen von ... °C | | | |
|---|---|---|---|---|
| gem. Bsp. | 220 | 260 | 300 | 10 min. bei 300 °C |
| 14 | 0,63 | 2,53 | 7,74 | 12,64 |
| 15 | 1,42 | 3,95 | 8,06 | 13,27 |
| Vergleich+) | 1,26 | 4,58 | 20,54 | 49,61 |
| Vergleich++) | 0,0 | 1,11 | 9,48 | 58,46 |

+) Stabilisator nach Bsp. 31 der DE-OS 26 06 026

++) Stabilisator nach Bsp. 1 der DE-OS 27 19 131

Beispiel 35

Dieses Beispielsoll die lichtstabilisierende Wirkung der neuen Verbindungen in Poly-α-Olefinen aufzeigen.

100 Gewichtsteile Polypropylen mit dem Schmelzindex $i_5$ (230 °C) von ca. 6 g/10 Min. (bestimmt nach ASTM D 1238-62 T) und einer Dichte von 0,90 wurden mit

0,1 Gew.-T. Pentaerythrityl-tetrakis-3-(3,5-di-tert.-butyl-4-hydroxiphenyl)-propionat,
0,2 Gew.-T. Calciumstearat und
0,1 Gew.-T. des zu prüfenden erfindungsgemäßen Stabilisators

vermischt.

Um eine möglichst gleichmäßige Verteilung auf dem Polymerkorn zu erreichen, wurden die Stabilisatoren in einem Lösemittel gelöst, und die Lösung wurde unter Rühren in das Polypropylenpulver eingetropft, wobei durch gleichzeitige Bestrahlung mit einer IR-Lampe der größte Teil des Lösemittels wieder abdampfte.

Nach ca. 20 Minuten wurde das Calciumstearat hinzugegeben und noch weitere 10 Minuten gemischt. Lösemittelreste wurden durch Trocknen bei 50 °C/120 Min. in Trockenschrank entfernt.

Das Polypropylen wurde auf einer Windsor-Spritzgußmaschine der Type SP 50 bei 240 °C zu 60 × 60 × 1 mm-Platten verspritzt. Aus diesen Platten wurden Prüfkörper nach DIN 53 455, Form 3, verkleinert im Maßstab 1 : 3, ausgestanzt. Die als Vergleichsmuster benötigten Prüfkörper wurden analog, jedoch unter Fortlassen des zu testenden Stabilisators bzw. unter Zusatz der Vergleichsstabilisatoren, hergestellt.

Zur Bestimmung der Lichtstabilität wurden die Proben in einer Xenotest-1200-Apparatur der Firma Orginal Hanau Quarzlampen GmbH der Bestrahlung mit Wechsellicht unterworfen. Die Strahlungsintensität wurde durch UV-Filter (Spezialfilterglas d = 1,7 mm) moduliert. Die Lichtbeständigkeit wurde nach DIN 53 387 (17 min Trockenperiode, 3 min beregnen, Schwarztafeltemperatur 45 °C, relative Luftfeuchtigkeit während der Trockenperiode 70 bis 75 %) geprüft. Gemessen wurde die Reißdehnung auf einer Zugprüfmaschine der Firma Instron bei einer Abzugsgeschwindigkeit von 5 cm/min nach einer bestimmten Belichtungszeit in Stunden.

| Stabilisator nach Bsp. | Belichtungszeit in Stunden | ermittelte Reißdehnung in % vom Ausgangswert |
|---|---|---|
| 14 | 1100 | 35 |
| 15 | 1100 | 40 |
| Polypropylen | 260 | 1 |
| Vergleich[1)] | 320 | 1 |
| Vergleich[2)] | 600 | 1 |
| Vergleich[3)] | 1100 | 1 |

[1)] ohne Lichtstabilisator
[2)] Verbindung nach Beispiel 1 der DE-OS 27 19 131
[3)] Verbindung nach Beispiel 31 der DE-OS 26 06 026

## Beispiel 36

In einem Laborschnellmischer wird aus Polypropylenpulver (®Hostalen PPU VP 1770 F der Hoechst AG) von Schmelzindex MFI 190/3 = 1,9 g/10 min s. DIN 53 535 und den unten angegebenen Rezepturbestandteilen eine homogene Mischung hergestellt und in ein Granulat überführt. Das so stabilisierte Material wird sodann in einem Laborextruder unter den üblichen Verarbeitungsbedingungen aufgeschmolzen und über eine Spinnpumpe mit Achtfachspinnkopf zu Monofilamenten verarbeitet, welche anschließend im Verhältnis 1 : 3 nachverstreckt, zu Garn von 40 dtex texturiert und zu Prüfgeweben verarbeitet werden.

100 Gew.-T. Polypropylen,
0,2 Gew.-T. Calciumstearat,
0,1 Gew.-T. 3,3-Bis-(3-tert.-butyl-4-hydroxiphenyl)-butansäureethylenglykolester,
0,1 Gew.-T. Dioctadecyldisulfid,
0,3 Gew.-T. des zu prüfenden Stabilisators

Die Gewebeproben werden auf einen gelochten Karton so aufgespannt, daß eine freie Öffnung von ca. 15,5 mm Durchmesser bestehen bleibt. In dieser Form werden die Prüflinge im Xenotest X 1 200 nach dem vorhergehenden Beispiel belichtet. In bestimmten Zeitabständen werden die Gewebe zentrisch mit einem Gewicht von 6 mm Durchmesser und einem Druck von 0,1 N/mm² belastet. Als Versagenstermin gilt das Durchbrechen des Gewichts.

| Stabilisator nach Beispiel | Belichtungszeit in Stunden beim Durchbruch des Gewichts |
|---|---|
| 14 | > 3100[4] |
| 15 | > 3100[4] |
| Polypropylen | 280 |
| Vergleich[1] | 400 |
| Vergleich[2] | 1400 |
| Vergleich[3] | 3000 |

[1]) ohne Stabilisator
[2]) Verbindung nach Bsp. 1 der DE-OS 27 19 131 (Handelsprodukt Tinuvin 622)
[3]) Verbindung nach Bsp. 31 der DE-OS 26 06 026
[4]) Gewicht noch nicht durchbrochen

Beispiel 37

Das wie im vorhergehenden Beispiel hergestellte stabilisierte Granulat wird auf einer Labor-Folienblasanlage (Schneckendurchmesser 25 mm, Länge 20 D), Temperaturprogramm 200, 240, 250, 255 °C) zu Blasfolien von ca. 70 µm Dichte verarbeitet. Diese Folien werden im Xenotest X 1 200 wie beschrieben künstlich bewittert. Als Schädigungsmerkmal wird die Carbonylzahl in Anlehnung an DIN 53 383, Teil 2, gemessen. (Diese wird für PP definiert als das Verhältnis der Extinktionen bei 1 715 $cm^{-1}$ und 1 524 $cm^{-1}$. Bei einer CO-Zahl > 2 beginnt der Zerfall der Prüfkörper zu Pulver.)

| Stabilisator | C=O-Zahl nach ... Stunden | | | |
|---|---|---|---|---|
| nach Beispiel | 500 | 1000 | 2000 | 2500 |
| 14 | 0,1 | 0,2 | 0,9 | 1,2 |
| 15 | 0,1 | 0,2 | 0,8 | 1 |
| Polypropylen | >2 | – | – | – |
| Vergleich[1] | >2 | – | – | – |
| Vergleich[2] | – | >2 | – | – |
| Vergleich[3] | 0,2 | 0,2 | 2 | – |

[1]), [2]) und [3]) entsprechen den Vergleichsproben des vorigen Beispiels

Beispiel 38

100 g eines thermosetting-Acrylat-Klarlacks (TSA) mit einem Feststoffanteil von 40 Gew.-% wurden mit 0,2 g des Stabilisators vermischt. Die Lösung wurde visuell auf Verträglichkeit geprüft.

| Stabilisator nach Beispiel | Löslichkeit |
|---|---|
| 14 | klar gelöst, homogen |
| 15 | klar gelöst, homogen |
| Vergleich[3] | trüb, inhomogen |

[3]) entsprechend Vergleichsprobe 3 des Beispiels 36.

**Ansprüche** (für die Vertragsstaaten : BE, CH, DE, FR, GB, IT, LI, NL, SE)

1. Verbindungen der allgemeinen Formel (I)

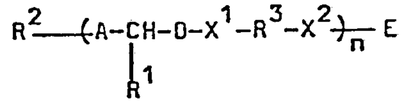 (I)

worin

n eine ganze Zahl von 1 bis 100 ist,

$X^1$ und $X^2$ entweder gleich sind und für eine Bindung, oder für

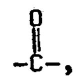, oder für 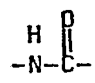

mit an den Stickstoff gebundenem $R^3$ stehen, oder, wenn E ohne Bedeutung und n = 1 ist, verschieden sind, wobei $X^2$ dann ohne Bedeutung ist,

$R^1$ Wasserstoff, $C_1$- bis $C_{30}$-Alkyl, Phenyl oder eine Gruppe der Formel (IIa) oder (IIb) ist,

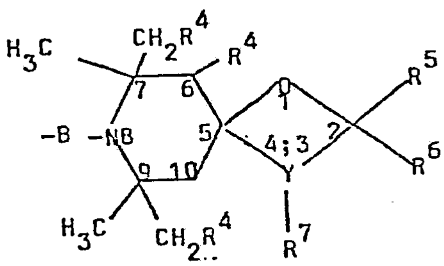

(IIa)

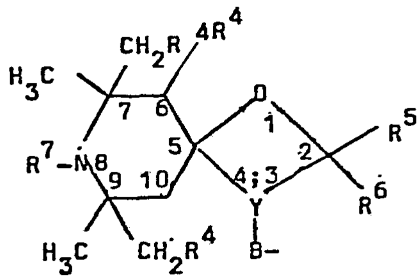

(IIb)

wobei in diesen Formeln

Y eine Radikal

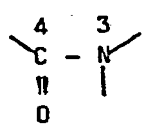 oder 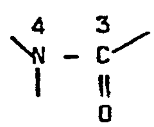

sein soll, das die Ringpositionen 3 ; 4 ; einnimmt,

B eine Bindung oder Methylen ist,

$R^4$ Wasserstoff oder Methyl bedeutet,

$R^5$ und $R^6$ gleich oder verschieden sind und Wasserstoff, $C_1$- bis $C_{18}$-Alkyl, unsubstituiertes oder durch Chlor oder $C_1$- bis $C_4$-Alkyl substituiertes Phenyl, unsubstituiertes oder auch $C_1$- bis $C_4$-Alkyl substituiertes $C_7$- bis $C_{14}$-Aralkyl bedeuten oder auch zusammen mit dem diese Reste bindenden Kohlenstoffatom einen unsubstituierten oder durch bis zu vier $C_1$- bis $C_4$-Alkylgruppen substituierten $C_5$- bis $C_{12}$-Cycloalkylring oder einen Piperidinring bilden,

$R^7$ ein Wasserstoffatom oder eine $C_1$- bis $C_4$-Alkylgruppe bedeutet, und

$R^2$ Wasserstoff, oder eine Gruppe der Formeln (II), oder $C_1$- bis $C_{30}$-Alkyl, oder Phenyl, oder eine durch $C_1$- bis $C_{18}$-Alkyl, $C_5$- oder $C_6$-Cycloalkyl oder Phenyl substituierte 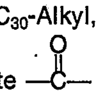 oder 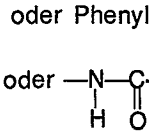-Gruppe mit an A gebundenem 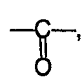, oder $C_2$- bis $C_{18}$-Alkylen, oder unsubstituiertes oder durch bis zu zwei $C_1$- bis $C_4$-Alkylgruppen substituiertes Phenylen, oder $C_7$- bis $C_{14}$-Aralkylen, oder α,ω-Dicarboxi-$C_1$- bis $C_8$-Alkylen oder ein Dicarboxi-$C_6$-Ring, oder ein Rest $>$N-Alkyl mit 1 bis 6 C-Atomen, oder eine Gruppe der Formel (III)

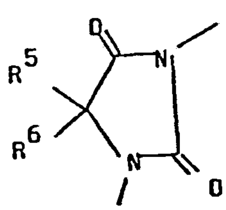 (III)

in welcher $R^5$ und $R^6$ die oben angegebenen Bedeutungen haben, oder ein Isocyanursäurerest, oder ein Stickstoffatom oder ein

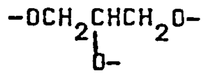 Rest ist,

$R^3$ wenn $X^1$, $X^2$ und E ohne Bedeutung sind, für Wasserstoff, oder wenn $X^2$ und E ohne Bedeutung sind für $C_1$- bis $C_{18}$-Alkyl, unsubstituiertes oder durch Chlor, Hydroxi, Amino oder $C_1$- bis $C_4$-Alkyl substituiertes Phenyl, $C_7$- bis $C_{14}$-Aralkyl, $C_5$- oder $C_6$-Cycloalkyl steht, oder die Bedeutung von $C_2$- bis $C_{12}$-Alkylen, oder von unsubstituiertem oder durch eine bis vier $C_1$- bis $C_4$-Alkylgruppen substituiertem Phenylen, oder von $C_7$- bis $C_{14}$-Aralkylen hat,

A für eine Bindung oder für $—CH_2—$, oder für $—OCH_2—$ mit an $R^2$ gebundenem —O—, oder für eine Gruppe der Formel (IV),

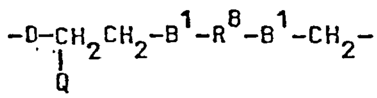 (IV)

in welcher Q für einen Rest (IIa) oder (IIb) steht, $B^1$ eine Bindung oder —O— ist und $R^8$ die Bedeutung von $R^2$ hat, wobei freie Valenzen durch die Gruppe

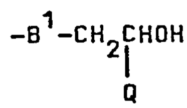

abgesättigt sind, oder für eine Gruppe der Formel (Va),

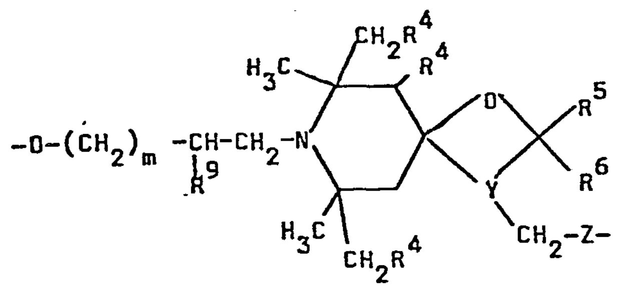 (Va)

in welcher $R^4$, $R^5$, $R^6$ und Y die oben angegebenen Bedeutungen haben, Z eine Bindung oder —CH(OH)— ist und $R^9$ für eine OH-Gruppe steht, sowie die Bedeutungen von $R^1$ hat, oder für eine Gruppe der Formeln (Vb) oder (Vc)

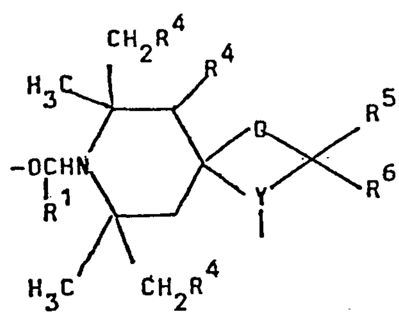

(Vb)

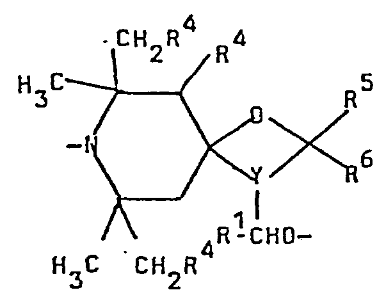

(Vc)

mit an $R^2$ gebundenem Sauerstoff, worin $R^1$, $R^4$, $R^5$, $R^6$ und Y die oben angegebenen Bedeutungen haben, steht, in oligomeren oder polymeren Produkten mit $n > 1$ $R^2$ als Endgruppe Wasserstoff, $R^3$ ein di- oder tri-funktioneller Rest, aus der für $R^3$ angegebenen Gruppe und A ein bifunktioneller Rest aus der unter A angegebenen Gruppe ist, und

E eine Gruppe der Formel

$$R^2—A—CH(R^1)—O—,$$

worin $R^1$, $R^2$ und A die oben angegebenen Bedeutungen haben, oder E ohne Bedeutung ist und

mindestens einer der Reste $R^1$, $R^2$ oder A ein Diazaspirodecansystem der Formeln (II) oder (V) enthält, oder E eine Endgruppe, die Niedrigalkylester in Form des Methyl- oder Ethylesters, —NCO, oder Halogen sein kann, ist.

2. Verfahren zur Herstellung von Verbindungen der Formel (I), dadurch gekennzeichnet, daß man entweder

a) ein Diazaspirodecan der allgemeinen Formel (VIa) bzw. (VIb)

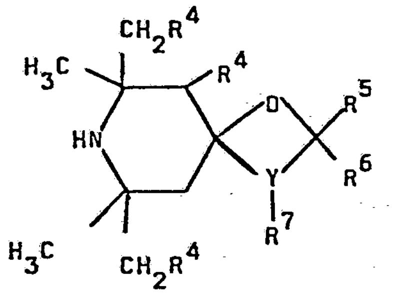

(VIa)

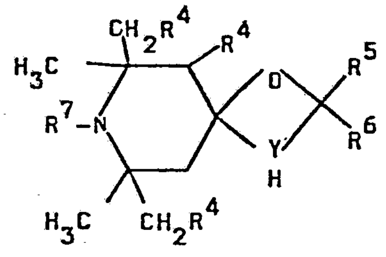

(VIb)

worin $R^4$, $R^5$, $R^6$, $R^7$ und Y die in Anspruch 1 angegebenen Bedeutungen haben in Gegenwart eines Katalysators bei erhöhten Temperaturen in einem organischen Lösungsmittel mit der 1,0 bis 1,5 fach valenten Menge eines Epoxids der Formel (VIIa), oder der 1,0 bis 3 fach valenten Menge eines Aldehyds der Formel (VIIb)

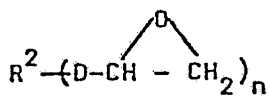

(VIIa)

$$R^2—(CHO)_n$$

(VIIb)

umsetzt, wobei $R^2$ in Formel (VIIb) die in Anspruch 1 angegebene Bedeutung und in Formel (VIIa) zusätzlich die einer Gruppe der Formeln (II) hat, n = 1, 2 oder 3 ist und D für eine Bindung, oder für Methylen oder für $—OCH_2—$ mit an $R^2$ gebundenem —O— steht, worauf man die so erhaltenen Alkohole gewünschtenfalls zur Herstellung von Folgeprodukten mit definiertem Molgewicht ebenfalls in Gegenwart eines Katalysators und gegebenenfalls eines Säureacceptors bei erhöhten Temperaturen in einem Lösungsmittel mit der äquivalenten, zur Herstellung von oligomeren oder polymeren Folgeprodukten jedoch mit der äquimolaren Menge einer bifunktionellen Verbindung der Formel (VIII)

$$R^3—(T)_m \qquad \text{(VIII)}$$

in welcher $R^3$ die in Anspruch 1 angegebene Bedeutung hat, m = 1, 2 oder 3 ist und T für Halogen, Niedrigalkylester, —COCl, —OH oder —NCO steht, umsetzt, oder

b) eine Verbindung der Formel (VIIa) mit n = 1, D = $—CH_2—$ und $R^2$ = eine Gruppe der Formel (IIa) oder (IIb) in der B eine Bindung darstellt, mit Ameisensäure, die in der 3 bis 10 fach molaren Menge anwesend sein soll und gleichzeitig als Lösungsmittel dient, bei 100 °C umsetzt, den gebildeten Ester sodann alkalisch verseift, und den derart erhaltenen Alkohol gewünschtenfalls wie oben angegeben, unter Zugabe einer Verbindung der Formel (VIII) einer Oligomerisierung oder einer Polymerisation unterwirft.

3. Verwendung der Verbindungen nach Anspruch 1 zum Stabilisieren von synthetischen Polymeren.

4. Verwendung nach Anspruch 3, dadurch gekennzeichnet, daß das Polymere eine Polyolefin, ein Polyacrylat oder Polymethacrylat, oder ein Homo- oder Copolymerisat des Styrols ist.

5. Verwendung nach Anspruch 3, dadurch gekennzeichnet, daß das Polymere den Feststoffanteil eines Lackes darstellt.

6. Verfahren zum Stabilisieren von synthetischen Polymeren gegen den schädigen Einfluß von Licht, dadurch gekennzeichnet, daß man den Polymeren, gegebenenfalls neben bisher bekannten, stabilisierend wirkendan Stoffen, 0,01 bis 5 Gewichtsteile, bezogen auf Polymeres, eines Stabilisators nach Anspruch 1 zusetzt.

7. Gegen UV-Zersetzung stabilisierte, synthetische Polymere, in denen 0,01 bis 5 Gewichtsteile, bezogen auf Polymeres, eines Stabilisators nach Anspruch 1 enthalten sind.

**Ansprüche** (für den Vertragsstaat AT)

1. Verfahren zur Herstellung von Verbindungen der allgemeinen Formel (I)

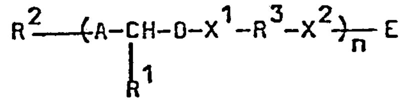 (I),

worin

n eine ganze Zahl von 1 bis 100 ist,

$X^1$ und $X^2$ entweder gleich sind und für eine Bindung, oder für

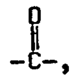 oder für 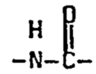

mit an den Stickstoff gebundenem $R^3$ stehen, oder, wenn E ohne Bedeutung und n = 1 ist, verschieden sind, wobei $X^2$ dann ohne Bedeutung ist,

$R^1$ Wasserstoff, $C_1$- bis $C_{30}$-Alkyl, Phenyl oder eine Gruppe der Formel (IIa) oder (IIb) ist,

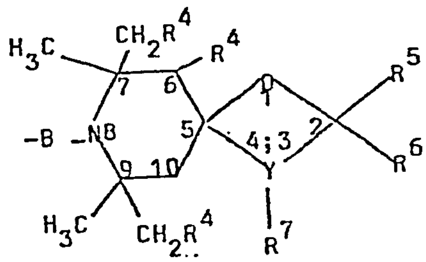

(IIa)

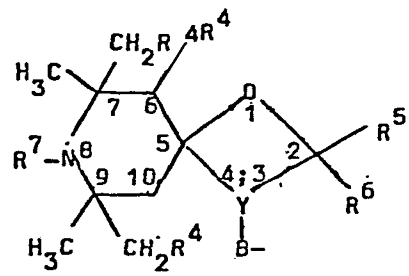

(IIb)

wobei in diesen Formeln

Y eine Radikal 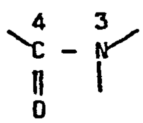 oder 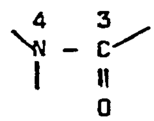

sein soll, das die Ringpositionen 3 ; 4 ; einnimmt,

B eine Bindung oder Methylen ist,

$R^4$ Wasserstoff oder Methyl bedeutet,

$R^5$ und $R^6$ gleich oder verschieden sind und Wasserstoff, $C_1$- bis $C_{18}$-Alkyl, unsubstituiertes oder durch Chlor oder $C_1$- bis $C_4$-Alkyl substituiertes Phenyl, unsubstituiertes oder auch $C_1$- bis $C_4$-Alkyl substituiertes $C_7$- bis $C_{14}$-Aralkyl bedeuten oder auch zusammen mit dem diese Reste bindenden Kohlenstoffatom einen unsubstituierten oder durch bis zu vier $C_1$- bis $C_4$-Alkylgruppen substituierten $C_5$- bis $C_{12}$-Cycloalkylring oder einen Piperidinring bilden,

$R^7$ ein Wasserstoffatom oder eine $C_1$- bis $C_4$-Alkylgruppe bedeutet, und

$R^2$ Wasserstoff, oder eine Gruppe der Formeln (II), oder $C_1$- bis $C_{30}$-Alkyl, oder Phenyl, oder eine durch $C_1$- bis $C_{18}$-Alkyl, $C_5$- oder $C_6$-Cycloalkyl oder Phenyl substituierte 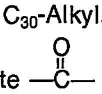 oder 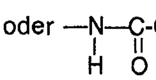-Gruppe mit an A gebundenem 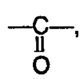, oder $C_2$- bis $C_{18}$-Alkylen, oder unsubstituiertes oder durch bis zu zwei $C_1$- bis $C_4$-Alkylgruppen substituiertes Phenylen, oder $C_7$- bis $C_{14}$-Aralkylen, oder α,ω-Dicarboxi-$C_1$- bis $C_8$-Alkylen oder ein Dicarboxi-$C_6$-Ring, oder ein Rest $\geq$N-Alkyl mit 1 bis 6 C-Atomen, oder eine Gruppe der Formel (III)

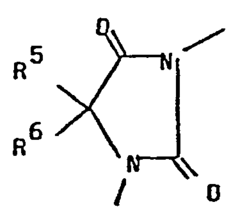 (III)

in welcher $R^5$ und $R^6$ die oben angegebenen Bedeutungen haben, oder ein Isocyanursäurerest, oder ein Stickstoffatom oder ein

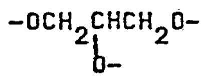 Rest ist,

$R^3$ wenn $X^1$, $X^2$ und E ohne Bedeutung sind, für Wasserstoff, oder wenn $X^2$ und E ohne Bedeutung sind für $C_1$- bis $C_{18}$-Alkyl, unsubstituiertes oder durch Chlor, Hydroxi, Amino oder $C_1$- bis $C_4$-Alkyl substituiertes Phenyl, $C_7$- bis $C_{14}$-Aralkyl, $C_5$- oder $C_6$-Cycloalkyl steht, oder die Bedeutung von $C_2$- bis $C_{12}$-Alkylen, oder von unsubstituiertem oder durch eine bis vier $C_1$- bis $C_4$-Alkylgruppen substituiertem Phenylen, oder von $C_7$- bis $C_{14}$-Aralkylen hat,

A für eine Bindung oder für $—CH_2—$, oder für $—OCH_2—$ mit an $R^2$ gebundenem —O—, oder für eine Gruppe der Formel (IV)

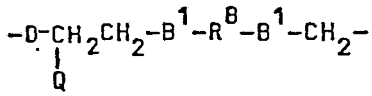

(IV)

in welcher Q für einen Rest (IIa) oder (IIb) steht, $B^1$ eine Bindung oder —O— ist und $R^8$ die Bedeutung von $R^2$ hat, wobei freie Valenzen durch die Gruppe

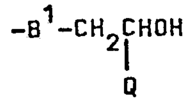

abgesättigt sind, oder für eine Gruppe der Formel (Va)

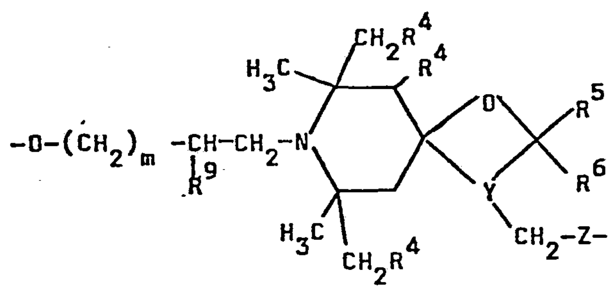

(Va)

in welcher $R^4$, $R^5$, $R^6$ und Y die oben angegebenen Bedeutungen haben, Z eine Bindung oder —CH(OH)— ist und $R^9$ für eine OH-Gruppe steht, sowie die Bedeutungen von $R^1$ hat, oder für eine Gruppe der Formeln (Vb) oder (Vc)

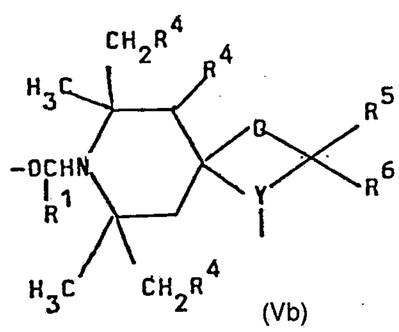

(Vb)

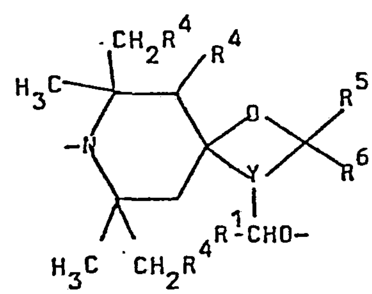

(Vc)

mit an $R^2$ gebundenem Sauerstoff, worin $R^1$, $R^4$, $R^5$, $R^6$ und Y die oben angegebenen Bedeutungen haben, steht, in oligomeren oder polymeren Produkten mit $n > 1$ $R^2$ als Endgruppe Wasserstoff, $R^3$ ein di- oder tri-funktioneller Rest, wie für n = 2, 3 angegeben ist, und A die für n = 2, 3 angegebenen Bedeutungen hat, und

E eine Gruppe der Formel

$$R^2—A—CH(R^1)—O—,$$

worin $R^1$, $R^2$ und A die oben angegebenen Bedeutungen haben, oder ohne Bedeutung ist und mindestens einer der Reste $R^1$, $R^2$ oder A ein Diazaspirodecansystem der Formeln (II) oder (V) enthält, oder eine Endgruppe, die Niedrigalkylester in Form des Methyl- oder Ethylesters, —NCO, oder Halogen sein kann, ist,

dadurch gekennzeichnet, daß man entweder

a) ein Diazaspirodecan der allgemeinen Formel (VIa) bzw. (VIb)

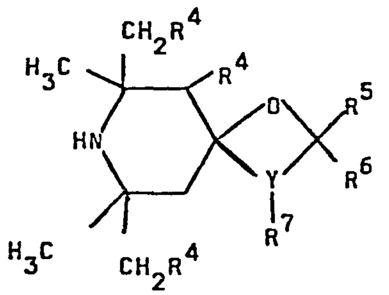

(VIa)

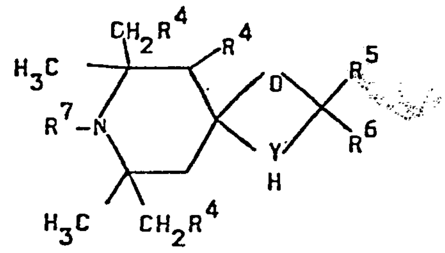

(VIb)

worin $R^4$, $R^5$, $R^6$, $R^7$ und Y die in Anspruch 1 angegebenen Bedeutungen haben in Gegenwart eines Katalysators bei erhöhten Temperaturen in einem organischen Lösungsmittel mit der 1,0 bis 1,5 fach valenten Menge eines Epoxids der Formel (VIIa), oder der 1,0 bis 3 fach valenten Menge eines Aldehyds der Formel (VIIb)

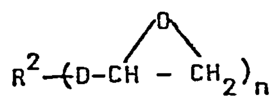

(VIIa)

$$R^2 \text{—} (CHO)_n$$

(VIIb)

umsetzt, wobei $R^2$ in Formel (VIIb) die in Anspruch 1 angegebene Bedeutung und in Formel (VIIa) zusätzlich die einer Gruppe der Formeln (II) hat, n = 1, 2 oder 3 ist und D für eine Bindung, oder für Methylen oder für $—OCH_2—$ mit an $R^2$ gebundenem —O— steht,
worauf man die so erhaltenen Alkohole gewünschtenfalls zur Herstellung von Folgeprodukten mit definiertem Molgewicht ebenfalls in Gegenwart eines Katalysators und gegebenenfalls eines Säureacceptors bei erhöhten Temperaturen in einem Lösungsmittel mit der äquivalenten, zur Herstellung von oligomeren oder polymeren Folgeprodukten jedoch mit der äquimolaren Menge einer bifunktionellen Verbindung der Formel (VIII)

$$R^3—(T)_m \qquad \text{(VIII)}$$

in welcher $R^3$ die in Anspruch 1 angegebene Bedeutung hat, m = 1, 2 oder 3 ist und T für Halogen, Niedrigalkylester, —COCl, —OH oder —NCO steht, umsetzt, oder

b) eine Verbindung der Formel (VIIa) mit n = 1, D = $—CH_2—$ und $R^2$ = eine Gruppe der Formel (IIa) oder (IIb) in der B eine Bindung darstellt, mit Ameisensäure, die in der 3 bis 10 fach molaren Menge anwesend sein soll und gleichzeitig als Lösungsmittel dient, bei 100 °C umsetzt, den gebildeten Ester sodann alkalisch verseift, und den derart erhaltenen Alkohol gewünschtenfalls wie oben engegeben, unter Zugabe einer Verbindung der Formel (VIII) einer Oligomerisierung oder einer Polymerisation unterwirft.

2. Verwendung der nach Anspruch 1 erhaltenen Verbindungen der allgemeinen Formel (I) zum Stabilisieren von synthetischen Polymeren.

3. Verwendung nach Anspruch 2, dadurch gekennzeichnet, daß das Polymere ein Polyolefin, ein Polyacrylat oder Polymethacrylat, oder ein Homo- oder Copolymerisat des Styrols ist.

4. Verwendung nach Anspruch 2, dadurch gekennzeichnet, daß das Polymere den Faststoffanteil eines Lackes darstellt.

5. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 5 Gewichtsteile, bezogen auf Polymeres, eines nach Anspruch 1 erhaltenen Stabilisators zusetzt.

6. Gegen UV-Zersetzung stabilisierte, synthetische Polymere, in denen 0,01 bis 5 Gewichtsteile, bezogen auf Polymeres, eines nach Anspruch 1 erhaltenen Stabilisators enthalten sind.

**Claims** (for the Contracting States : BE, CH, DE, FR, GB, IT, LI, NL, SE)

1. A compound of the general formula (I)

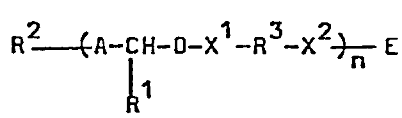

(I)

wherein

n is an integer from 1 to 100,

$X^1$ and $X^2$ are either identical and represent a bond or

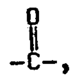 or 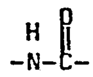

in which $R^3$ is attached to the nitrogen, or, if E has no meaning and n is 1, are different, in which case X2 then has no meaning,

$R^1$ is hydrogen, $C_1$ to $C_{30}$ alkyl, phenyl or a group of the formula (IIa) or (IIb)

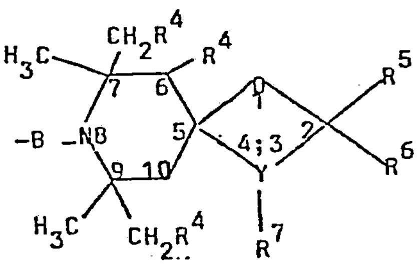

(IIa)

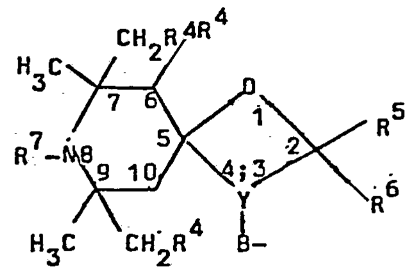

(IIb)

in which formulae

Y should be a radical

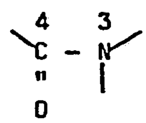 or 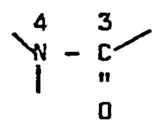

which occupies the ring positions 3 ; 4,

B is a bond or is methylene,

$R^4$ denotes hydrogen or methyl,

$R^5$ and $R^6$ are identical or different and denote hydrogen, $C_1$ to $C_{18}$ alkyl, phenyl which is unsubstituted or substituted by chlorine or $C_1$ to $C_4$ alkyl, or $C_7$ to $C_{14}$ aralkyl which is unsubstituted or substituted by $C_1$ to $C_4$ alkyl, or, together with the carbon atom linking these radicals, form a $C_5$ to $C_{12}$ cycloalkyl ring which is unsubstituted or substituted by up to four $C_1$ to $C_4$ alkyl groups, or form a piperidine ring,

$R^7$ denotes y hydrogen atom or a $C_1$ to $C_4$ alkyl group and

$R^2$ is hydrogen or a group of the formulae (II) or $C_1$ to $C_{30}$ alkyl or phenyl or a 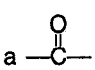 or 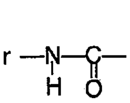 group which is substituted by $C_1$ to $C_{18}$ alkyl, $C_5$ or $C_6$ cycloalkyl or phenyl and which has 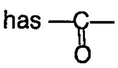 linked to A, or $C_2$ to $C_{18}$ alkylene, or phenylene which is unsubstituted or substituted by up to two $C_1$ to $C_4$ alkyl groups, or $C_7$ to $C_{14}$ aralkylene or α,ω-dicarboxy-$C_1$ to $C_8$ alkylene or a dicarboxy-$C_6$ ring or a radical >N-alkyl having 1 to 6 C atoms or a group of the formula (III)

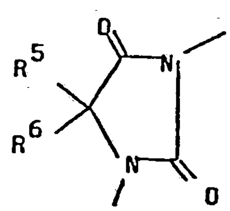

(III)

in which $R^5$ and $R^6$ have the meanings indicated above, or an isocyanuric acid radical or a nitrogen atom or a

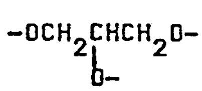 radical,

$R^3$ represents hydrogen if $X^1$, $X^2$ and E have no meaning, or, if $X^2$ and E have no meaning, represents $C_1$ to $C_{18}$ alkyl or phenyl, which is unsubstituted or substituted by chlorine, hydroxyl, amino or $C_1$ to $C_4$ alkyl, $C_7$ to $C_{14}$ aralkyl or $C_5$ or $C_6$ cycloalkyl or denotes $C_2$ to $C_{12}$ alkylene or phenylene which is unsubstituted or substituted by one to four $C_1$ to $C_4$ alkyl groups, or denotes $C_7$ to $C_{14}$ aralkylene,

A represents a bond or $-CH_2-$ or $-OCH_2-$ having —O— linked to $R^2$, or a group of the formula (IV)

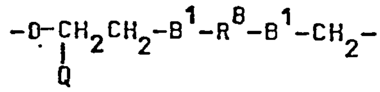

(IV)

in which Q represents a radical (IIa) or (IIb), $B^1$ is a bond or —O— and $R^8$ has the same meaning as $R^2$, free valences being saturated by the group

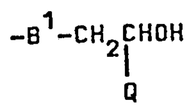

or A represents a group of the formula (Va)

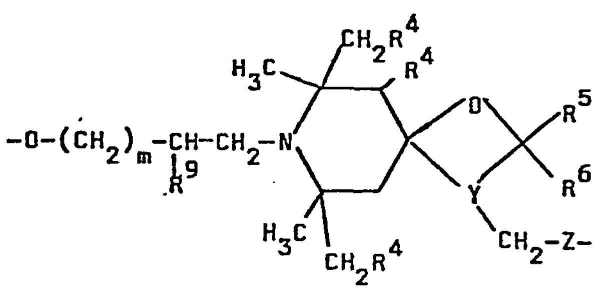

(Va)

in which $R^4$, $R^5$, $R^6$ and Y have the meanings indicated above, Z is a bond or —CH(OH)— and $R^9$ represents an OH group and has the meanings of $R^1$, or A represents a group of the formulae (Vb) or (Vc)

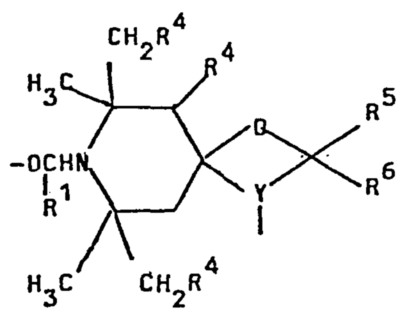

(Vb)

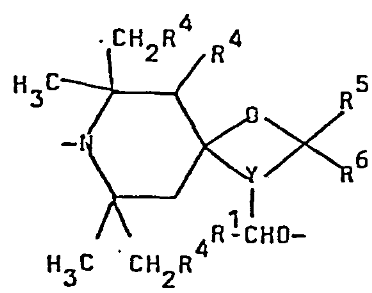

(Vc)

having oxygen attached to $R^2$, in which formulae $R^1$, $R^4$, $R^5$, $R^6$ and Y have the meanings indicated above, in oligomeric or polymeric products in which $n > 1$ $R^2$ as a terminal group, is hydrogen, $R^3$ is a bifunctional or trifunctional radical, from the group indicated for $R^3$, and A has the meaning of a bifunctional radical from the group indicated for A, and

E is a group of the formula

$$R^2-A-CH(R^1)-O-$$

wherein $R^1$, $R^2$ and A have the meanings indicated above, or E has no meaning, and at least one of the radicals $R^1$, $R^2$ or A contains a diazaspirodecane system of the formulae (II) or (V), or E is a terminal group which can be a lower alkyl ester in the form of the methyl- or ethyl ester, —NCO or halogen.

2. A process for the preparation of compounds of the formula (I), which comprises either

a) reacting a diazaspirodecane of the general formula (VIa) or (VIb)

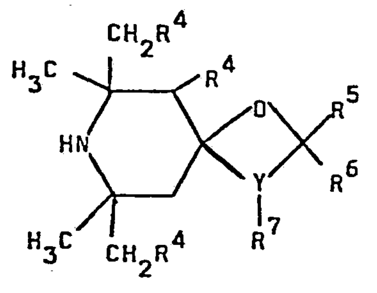

(VIa)

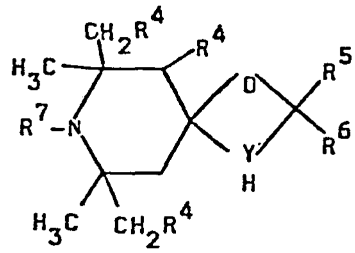

(VIb)

wherein $R^4$, $R^5$, $R^6$, $R^7$ and Y have the meanings indicated in claim 1, in the presence of a catalyst, at elevated temperatures and in an organic solvent, with 1.0 to 1.5 times the equivalent quantity of an epoxide of the formula (VIIa), or with 1.0 to 3 times the equivalent quantity of an aldehyde of the formula (VIIb)

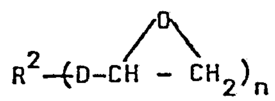

(VIIa)

$$R^2-(CHO)_n$$

(VIIb)

$R^2$ in formula (VIIb) having the meaning indicated in claim 1, and in formula (VIIa) having additionally the meaning of a group of the formulae (II), n being 1, 2 or 3 and D representing a bond or methylene or $-OCH_2-$ in which —O— is attached to $R^2$,

after which, if desired, in order to prepare secondary products having a definite molecular weight, the alcohols thus obtained are also reacted in the presence of a catalyst and, if appropriate, and acid acceptor, at elevated temperatures and in a solvent, with an equivalent quantity, but for the preparation of oligomeric or polymeric secondary products using an equimolar quantity, of a bifunctional compound of the formula (VIII)

$$R^3-(T)_m \qquad \text{(VIII)}$$

in which $R^3$ has the meaning indicated in claim 1, m is 1, 2 or 3 and T represents halogen, a lower alkyl ester, —COCl, —OH or —NCO, or

b) reacting a compound of the formula (VIIa) in which n = 1, D = $-CH_2-$ and $R^2$ = a group of the formula (IIa) or (IIb) in which B represents a bond, at 100 °C with formic acid, which should be present in 3 to 10 times the molar quantity and acts at the same time as the solvent, then subjecting the ester which has been formed to alkaline saponification and, if desired, subjecting the alcohol obtained in this way to an oligomerization or a polymerization, as indicated above, with the addition of a compound of the formula (VIII).

3. Use of the compounds as claimed in claim 1 for stabilizing synthetic polymers.

4. Use as claimed in claim 3, wherein the polymer is a polyolefin, a polyacrylate or polymethacrylate or a homopolymer or copolymer of styrene.

5. Use as claimed in claim 3, wherein the polymer constitutes the solid component of a lacquer.

6. A process for the stabilization of synthetic polymers against the harmful effect of light, which comprises adding to the polymers, if appropriate in addition to hitherto known substances having a stabilizing action, 0.01 to 5 parts by weight, relative to the polymer, of a stabilizer as claimed in claim 1.

7. Synthetic polymers which have been stabilized against UV decomposition and which contain 0.01 to 5 parts by weight, relative to the polymer, of a stabilizer as claimed in claim 1.

**Claims** (for the Contracting State AT)

1. A process for the preparation of compounds of the general formula (I)

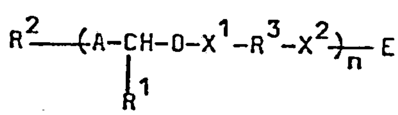

(I)

wherein

n is an integer from 1 to 100,

$X^1$ and $X^2$ are either identical and represent a bond or

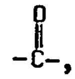, or 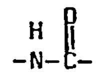

in which $R^3$ is attached to the nitrogen, or if E has no meaning and n is 1, are different, in which case $X^2$ then has no meaning,

$R^1$ is hydrogen, $C_1$ to $C_{30}$ alkyl, phenyl or a group of the formula (IIa) or (IIb)

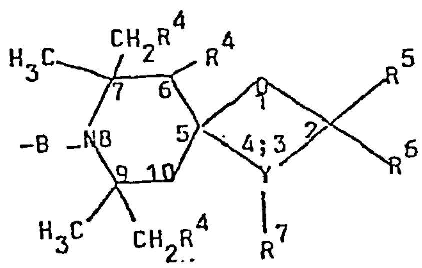

(IIa)

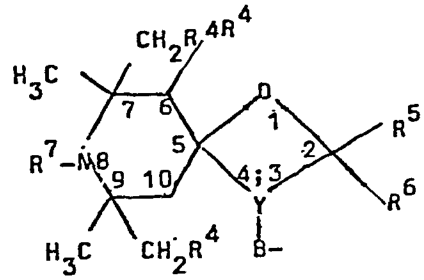

(IIb)

in which formulae

Y should be a radical

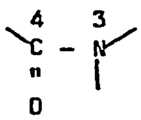 or 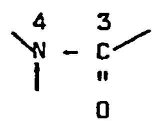

which occupies the ring positions 3 ; 4,

B is a bond or is methylene,

$R^4$ denotes hydrogen or methyl,

$R^5$ and $R^6$ are identical or different and denote hydrogen $C_1$ to $C_{18}$ alkyl, phenyl which is unsubstituted or substituted by chlorine or $C_1$ to $C_4$ alkyl, or $C_7$ to $C_{14}$ aralkyl which is unsubstituted or substituted by $C_1$ to $C_4$ alkyl, or together with the carbon atom lining these radicals, form a $C_5$ to $C_{12}$ cycloalkyl ring which is unsubstituted or substituted by up to four $C_1$ to $C_4$ alkyl groups, or form a piperidine ring,

$R^7$ denotes a hydrogen atom or a $C_1$ to $C_4$ alkyl group and

$R^2$ is hydrogen or a group of the formulae (II) or $C_1$ to $C_{30}$ alkyl or phenyl or a 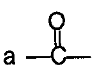 or 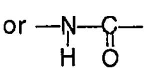 group which is substituted by $C_1$ to $C_{18}$ alkyl, $C_5$ or $C_6$ cycloalkyl or phenyl and which has 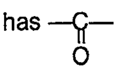 linked to A, or $C_2$ to $C_{18}$ alkylene, or phenylene which is unsubstituted or substituted by up to two $C_1$ to $C_4$ alkyl groups, or $C_7$ to $C_{14}$ aralkylene or α,ω-dicarboxy-$C_1$ to $C_8$ alkylene or a dicarboxy-$C_6$ ring or a radical >N-alkyl having 1 to 6 C atoms or a group of the formula (III)

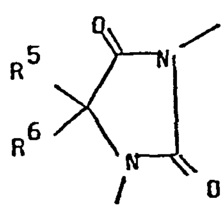

(III)

in which $R^5$ and $R^6$ have the meanings indicated above, or an isocyanuric acid radical or a nitrogen atom or a

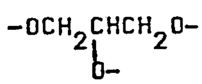 radical,

$R^3$ represents hydrogen if $X^1$, $X^2$ and E have no meaning, or, if $X^2$ and E have no meaning, represents $C_1$ to $C_{18}$ alkyl or phenyl, which is unsubstituted or substituted by chlorine, hydroxyl, amino or $C_1$ to $C_4$ alkyl, $C_7$ to $C_{14}$ aralkyl or $C_5$ or $C_6$ cycloalkyl or denotes $C_2$ to $C_{12}$ alkylene or phenylene which is

unsubstituted or substituted by one to four $C_1$ to $C_4$ alkyl groups, or denotes $C_7$ to $C_{14}$ aralkylene,

A represents a bond or $—CH_2—$ or $—OCH_2—$ having —O— linked to $R^2$, or a group of the formula (IV)

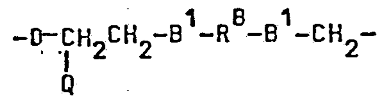

(IV)

in which Q represents a radical (IIa) or (IIb), $B^1$ is a bond or —O— and $R^8$ has the meanings as $R^2$, free valences being saturated by the group

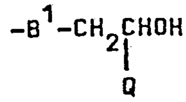

or A represents a group of the formula (Va)

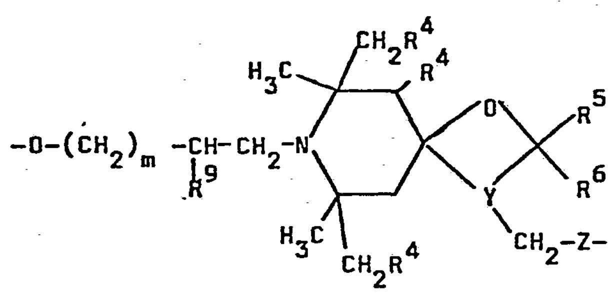

(Va)

in which $R^4$, $R^5$, $R^6$ and Y have the meanings indicated above, Z is a bond or —CH(OH)— and $R^9$ represents an OH group and has the meanings of $R^1$, or A represents a group of the formulae (Vb) or (Vc)

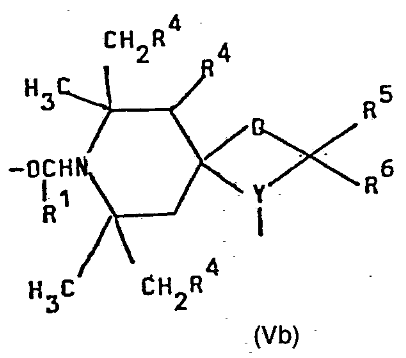

(Vb)

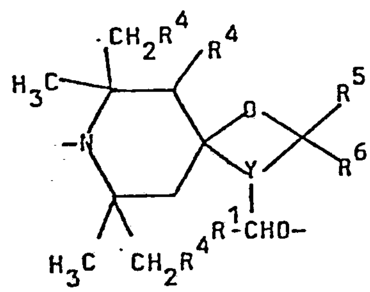

(Vc)

having oxygen attached to $R^2$, in which formulae $R^1$, $R^4$, $R^5$, $R^6$ and Y have the meanings indicated above, in oligomeric or polymeric products in which $n > 1$ $R^2$, as a terminal group, is hydrogen, $R^3$ is a bifunctional or trifunctional radical, as indicated for n = 2, 3, and A has the meanings as indicated for n = 2, 3, and E is a group of the formula

$$R^2—A—CH(R^1)—O—$$

wherein $R^1$, $R^2$ and A have the meanings indicated above, or E has no meaning, and at least one of the radicals $R^1$, $R^2$ or A contains a diazaspirodecane system of the formulae (II) or (V), or E is a terminal group which can be a lower alkyl ester in the form of the methyl- or ethyl ester —NCO or halogen, which comprises either

a) reacting a diazaspirodecane of the general formula (VIa) or (VIb)

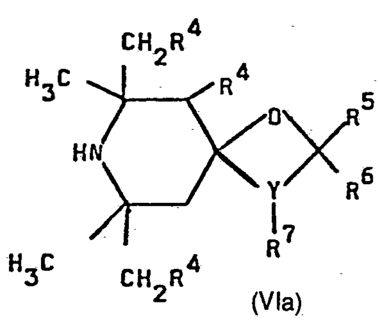

(VIa)

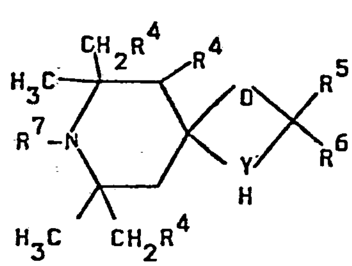

(VIb)

wherein $R^4$, $R^5$, $R^6$, $R^7$ and Y have the meanings indicated in claim 1, in the presence of a catalyst, at elevated temperatures and in an organic solvent, with 1.0 to 1.5 times the equivalent quantity of an epoxide of the formula (VIIa), or with 1.0 to 3 times the equivalent quantity of an aldehyde of the formula (VIIb)

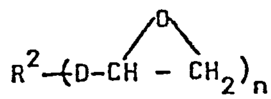

$$R^2\!-\!(CHO)_n$$

(VIIa) (VIIb)

$R^2$ in formula (VIIb) having the meaning indicated above, and in formula (VIIa) having additionally the meaning of a group of the formulae (II), n being 1, 2 or 3 and D representing a bond or methylene or —$OCH_2$— in which —O— is attached to $R^2$, after which, if desired, in order to prepare secondary products having a definite molecular weight, the alcohols thus obtained are also reacted in the presence of a catalyst and, if appropriate, and acid acceptor, at elevated temperatures and in a solvent, with an equivalent quantity, but, for the preparation of oligomeric or polymeric secondary products using an equimolar quantity, of a bifunctional compound of the formula (VIII)

$$R^3\!-\!(T)_m \qquad \text{(VIII)}$$

in which $R^3$ has the meaning indicated above, m is 1, 2 or 3 and T represents halogen, a lower alkyl ester, —COCl, —OH or —NCO, or

b) reacting a compound of the formula (VIIa) in which n = 1, D = —$CH_2$— and $R^2$ = a group of the formula (IIa) or (IIb) in which B represents a bond, at 100 °C with formic acid, which should be present in 3 to 10 times the molar quantity and acts at the same time as a solvent, then subjecting the ester which has been formed to alkaline saponification and, if desired, subjecting the alcohol obtained in this way to an oligomerization or a polymerization, as indicated above, with the addition of a compound of the formula (VIII).

2. Use of the compounds of the general formula (I) obtained according to claim 1 for stabilizing synthetic polymers.

3. Use as claimed in claim 2, wherein the polymer is a polyolefin, a polyacrylate or polymethacrylate or a homopolymer or copolymer of styrene.

4. Use as claimed in claim 2, wherein the polymer constitutes the solid component of a lacquer.

5. A process for the stabilization of synthetic polymers against the harmful effect of light, which comprises adding to the polymers, if appropriate in addition to hitherto known substances having a stabilizing action, 0.01 to 5 parts by weight, relative to the polymer, of a stabilizer obtained according to claim 1.

6. Synthetic polymers which have been stabilized against UV decomposition and which contain 0.01 to 5 parts by weight, relative to the polymer, of a stabilizer obtained according to claim 1.

**Revendications** (pour les Etats contractants : BE, CH, DE, FR, GB, IT, LI, NL, SE)

1. Composés de formule générale (I)

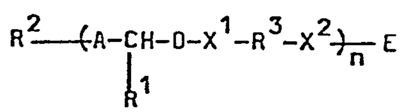

(I)

dans laquelle

n est un nombre entier valant 1 à 100,

$X^1$ et $X^2$ sont identiques et représentent une liaison, ou

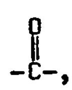 ou 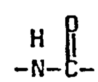

avec $R^3$ relié à l'azote ou bien, quand E n'a pas de signification et n vaut 1, ils sont différents et $X^2$ n'a alors pas de signification,

$R^1$ représente un atome d'hydrogène, un groupe alkyle en $C_1$ à $C_{30}$, un groupe phényle ou un groupe répondant aux formules (IIa) ou (IIb)

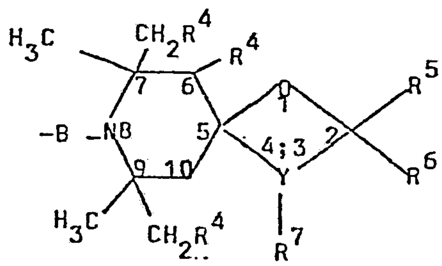

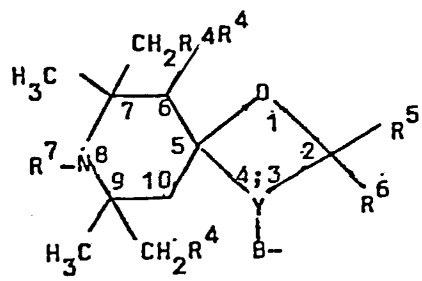

(IIa) (IIb)

dans lesquelles

Y représente un radical

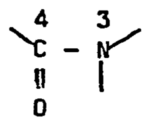 ou 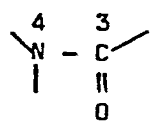

qui occupe les positions de cycle 3 ; 4

B représente une liaison ou un groupe méthylène,

$R^4$ représente un atome d'hydrogène ou un groupe méthyle,

$R^5$ et $R^6$ sont identiques ou différents et représentent de l'hydrogène, un groupe alkyle en $C_1$ à $C_{18}$, un groupe phényle non substitué ou substitué par du chlore ou par un groupe alkyle en $C_1$ à $C_4$, un groupe aralkyle en $C_7$ à $C_{14}$ non substitué ou substitué par un groupe alkyle en $C_1$ à $C_4$, ou bien pris ensemble avec l'atome de carbone reliant ces radicaux, ils forment un groupe cycloalkyle en $C_5$ à $C_{12}$ non substitué ou substitué par jusqu'à quatre groupes alkyles en $C_1$ à $C_4$, ou ils forment un groupe noyau pipéridine,

$R^7$ représente un atome d'hydrogène ou un groupe alkyle en $C_1$ à $C_4$, et

$R^2$ représente un atome d'hydrogène ou un groupe répondant aux formules (II), ou un groupe alkyle en $C_1$ à $C_{30}$ ou un groupe phényle ou un groupe 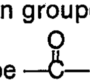 ou 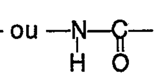 substitué par un groupe alkyle en $C_1$ à $C_{18}$ ou cycloalkyle en $C_5$ ou $C_6$, ou phényle avec 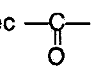 relié à A, ou bien un groupe alkylène en $C_2$ à $C_{18}$, ou un groupe phénylène non substitué ou substitué par jusqu'à deux groupes alkyles en $C_1$ à $C_4$, ou un groupe aralkylène en $C_7$ à $C_{14}$ ou un groupe dicarboxy-α,ω alkylène en $C_1$ à $C_8$ ou un noyau dicarboxy en $C_6$, ou un radical $\gtrdot$N-alkyle comportant 1 à 6 atomes de carbone, ou un groupe de formule (III)

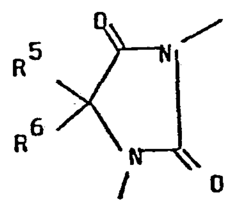

(III)

dans laquelle $R^5$ et $R^6$ ont les sens indiqués ci-dessus, ou un radical d'acide isocyanurique, ou un atome d'azote ou un radical

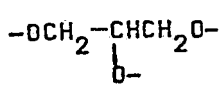

$R^3$ représente, quand $X^1$, $X^2$ et E n'ont pas de signification, un atome d'hydrogène ou bien, quand $X^2$ et E n'ont pas de signification, $R^3$ représente un groupe alkyle en $C_1$ à $C_{18}$, un groupe phényle non substitué ou substitué par du chlore, un groupe hydroxyle, amino ou alkyle en $C_1$ à $C_4$, un groupe aralkyle en $C_7$ à $C_{14}$, un groupe cycloalkyle en $C_5$ ou $C_6$, ou il représente un groupe alkylène en $C_2$ à $C_{12}$, ou un groupe phénylène non substitué ou substitué par jusqu'à quatre groupes alkyle en $C_1$ à $C_4$, ou un groupe aralkylène en $C_7$ à $C_{14}$,

A représente une liaison ou $-CH_2-$, ou $-OCH_2-$ avec $-O-$ relié à $R^2$, ou bien A représente un groupe de formule (IV)

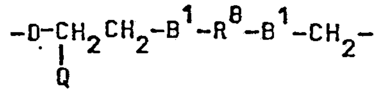

(IV)

dans laquelle Q représente un radical (IIa) ou (IIb), $B^1$ est une liaison ou représente —O— et $R^8$ a le sens de $R^2$, les valences libres étant saturées par le groupe

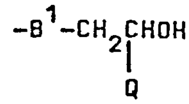

ou A représente un groupe de formule (Va)

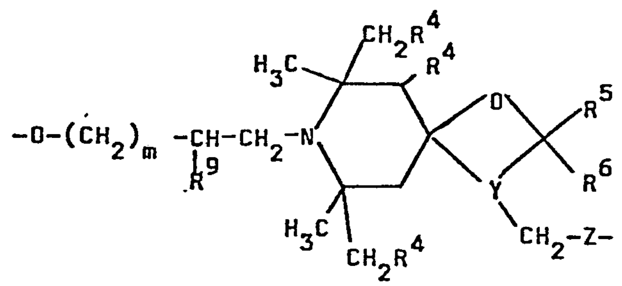

(Va)

dans laquelle $R^4$, $R^5$, $R^6$ et Y ont les sens indiqués ci-dessus, Z est une liaison ou représente —CH(OH)— et $R^9$ représente un groupe OH, et a les significations de $R^1$, ou bien A représente un groupe répondant aux formules (Vb) ou (Vc)

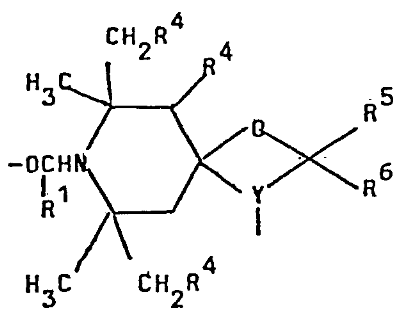

(Vb)

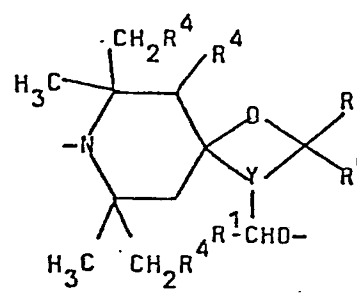

(Vc)

l'oxygène étant relié à $R^2$, et $R^1$, $R^4$, $R^5$, $R^6$ et Y ayant les sens indiqués ci-dessus, dans des produits oligomères ou polymères dans lesquels $n > 1$, $R^2$ représente comme groupe terminal de l'hydrogène, $R^3$ un radical di ou trifonctionnel, parmi les groupes indiqués pour $R^3$ et A est un groupe radical bifonctionnel pris parmi les groupes indiqués pour A, et représente un groupe de formule $R^2$—A—CH($R^1$)—O—, dans laquelle $R^1$, $R^2$ et A ont les sens indiqués ci-dessus, ou bien E n'a pas de signification et au moins l'un des radicaux $R^1$, $R^2$ ou A contiennent un système diazaspirodécane répondant aux formules (II) ou (V), ou bien E représente un groupe terminal qui peut être un groupe ester alkylique inférieur ayant la forme de l'ester méthylique ou éthylique, un groupe —NCO ou un halogène.

2. Procédé de préparation de composés de formule (I), caractérisé en ce que :

a) on fait réagir un diazaspirodécane répondant aux formules générales (VIa) ou (VIb)

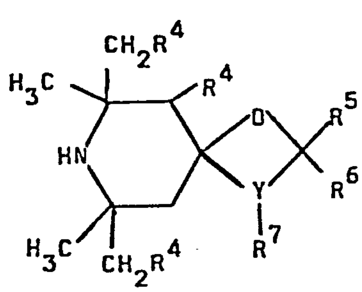

(VIa)

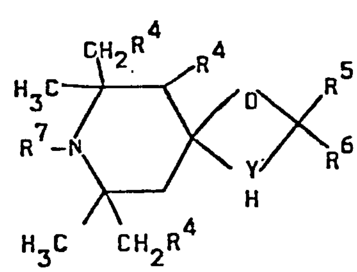

(VIb)

(dans lesquelles $R^4$, $R^5$, $R^6$, $R^7$ et Y ont les sens indiqués à la revendication 1)
en présence d'un catalyseur à des températures élevées dans un solvant organique avec 1,0 à 1,5 fois la

quantité équivalente d'un époxyde de formule (VIIa), ou bien 1,0 à 3 fois la quantité équivalente d'un aldéhyde de formule (VIIb) :

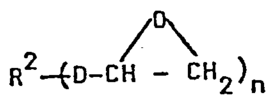

$R^2—(CHO)_n$

(VIIa) (VIIb)

$R^2$ ayant dans la formule (VIIb) le sens indiqué à la revendication 1 et pouvant en outre dans la formule (VIIa) être un groupe répondant aux formules (II), n vaut 1, 2 ou 3 et D représente une liaison, ou bien un groupe méthylène ou $—OCH_2—$ dont —O— est relié à $R^2$, puis on fait réagir les alcools ainsi obtenus, si l'on souhaite, pour préparer des produits suivants ayant un poids molaire défini, également en présence d'un catalyseur et éventuellement d'un accepteur d'acide, à des températures élevées dans un solvant, avec l'équivalent mais, pour préparer des produits suivants oligomères ou polymères, avec la quantité équimolaire d'un composé bifonctionnel de formule (VIII)

$$R^3—(T)_m \quad \text{(VIII)}$$

dans laquelle $R^3$ a le sens indiqué à la revendication 1, m vaut 1, 2 ou 3 et T représente un halogène, un groupe ester alkylique inférieur, —COCl, —OH ou —NCO, ou

b) on fait réagir à 100 °C un composé de formule (VIIa), dans laquelle n vaut 1, D représente $—CH_2—$ et $R^2$ est un groupe de formule (IIa) ou (IIb), dans laquelle B représente une liaison, avec l'acide formique, qui doit être présent en une quantité molaire triple à décuple, et sert en même temps de solvant, on saponifie ensuite en milieu alcalin l'ester formé et, si on le souhaite, on soumet l'alcool ainsi obtenu, comme indiqué ci-dessus, avec addition d'un composé de formule (VIII) à une oligomérisation ou à une polymérisation.

3. Utilisation des composés selon la revendication 1 pour stabiliser des polymères synthétiques.

4. Utilisation selon la revendication 3, caractérisée en ce que le polymère est une polyoléfine, un polyacrylate ou un polyméthacrylate, ou un homopolymère ou copolymère du styrène.

5. Utilisation selon la revendication 3, caractérisée en ce que le polymère constitue la partie solide d'un vernis ou d'une peinture.

6. Procédé pour stabiliser des polymères synthétiques contre l'influence endommageante de la lumière, procédé caractérisé en ce qu'on ajoute au polymère éventuellement en plus de substances connues jusqu'à présent et à rôle stabilisant, 0,01 à 5 parties en poids, par rapport au polymère, d'un stabilisant selon la revendication 1.

7. Polymères synthétiques stabilisés à l'encontre d'une décomposition par l'ultraviolet, et qui contiennent 0,01 à 5 parties en poids par rapport au polymère, d'un stabilisant selon la revendication 1.

**Revendications** (pour l'Etat contractant AT)

1. Procédé de préparation de composés de formule générale (I)

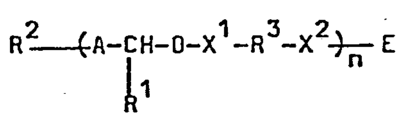

(I)

dans laquelle

n est un nombre entier valant 1 à 100,

$X^1$ et $X^2$ sont identiques et représentent une liaison, ou

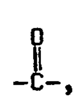 ou 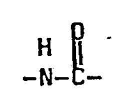

avec $R^3$ relié à l'azote ou bien, quand E n'a pas de signification et n vaut 1, ils sont différents et $X^2$ n'a alors pas de signification,

$R^1$ représente un atome d'hydrogène, un groupe alkyle en $C_1$ à $C_{30}$, un groupe phényle ou un groupe répondant aux formules (IIa) ou (IIb)

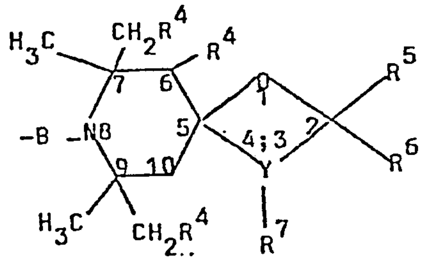

(IIa)

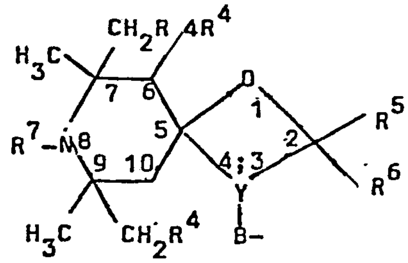

(IIb)

dans lesquelles

Y représente un radical

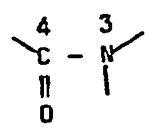

ou

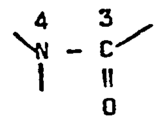

qui occupe les positions de cycle 3 ; 4

B représente une liaison ou un groupe méthylène,

$R^4$ représente un atome d'hydrogène ou un groupe méthyle,

$R^5$ et $R^6$ sont identiques ou différents et représentent de l'hydrogène, un groupe alkyle en $C_1$ à $C_{18}$, un groupe phényle non substitué ou substitué par du chlore ou par un groupe alkyle en $C_1$ à $C_4$, un groupe aralkyle en $C_7$ à $C_{14}$ non substitué ou substitué par un groupe alkyle en $C_1$ à $C_4$, ou bien pris ensemble avec l'atome de carbone reliant ces radicaux, ils forment un groupe cycloalkyle en $C_5$ à $C_{12}$ non substitué ou substitué par jusqu'à quatre groupes alkyles en $C_1$ à $C_4$, ou ils forment un noyau pipéridine,

$R^7$ représente un atome d'hydrogène ou un groupe alkyle en $C_1$ à $C_4$, et

$R^2$ représente un atome d'hydrogène ou un groupe répondant aux formules (II), ou un groupe alkyle en $C_1$ à $C_{30}$ ou un groupe phényle ou un groupe $-\overset{O}{\overset{\|}{C}}-$ ou $-\underset{H}{\underset{|}{N}}-\underset{O}{\underset{\|}{C}}-$ substitué par un groupe alkyle en $C_1$ à $C_{18}$, ou cycloalkyle en $C_5$ ou $C_6$, ou phényle avec $-\underset{O}{\underset{\|}{C}}-$ relié à A, ou bien un groupe alkylène en $C_2$ à $C_{18}$, ou un groupe phénylène non substitué ou substitué par jusqu'à deux groupes alkyles en $C_1$ à $C_4$, ou un groupe aralkylène en $C_7$ à $C_{14}$ ou un groupe dicarboxy-α,ω alkylène en $C_1$ à $C_8$ ou un noyau dicarboxylé en $C_6$, ou un radical $\geq$N-alkyle comportant 1 à 6 atomes de carbone, ou un groupe de formule (III)

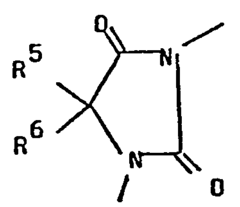

(III)

dans laquelle $R^5$ et $R^6$ ont les sens indiqués ci-dessus, ou un radical d'acide isocyanurique, ou un atome d'azote ou un radical

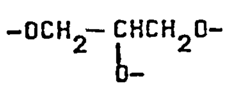

$R^3$ représente, quand $X^1$, $X^2$ et E n'ont pas de signification, un atome d'hydrogène ou bien, quand $X^2$ et E n'ont pas de signification, $R^3$ représente un groupe alkyle en $C_1$ à $C_{18}$, un groupe phényle non substitué ou substitué par du chlore, un groupe hydroxyle, amino ou alkyle en $C_1$ à $C_4$, un groupe aralkyle en $C_7$ à $C_{14}$, un groupe cycloalkyle en $C_5$ ou $C_6$, ou il représente un groupe alkylène en $C_2$ à $C_{12}$, ou un groupe phénylène non substitué ou substitué par jusqu'à quatre groupes alkyles en $C_1$ à $C_4$, ou un groupe aralkylène en $C_7$ à $C_{14}$,

A représente une liaison ou $-CH_2-$, ou $-OCH_2-$ avec $-O-$ relié à $R^2$, ou bien A représente un groupe de formule (IV)

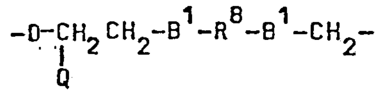

(IV)

dans laquelle Q représente un radical (IIa) ou (IIb), $B^1$ est une liaison ou représente —O— et $R^8$ a le sens de $R^2$, les valences libres étant saturées par le groupe

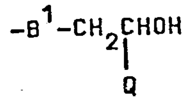

ou A représente un groupe de formule (Va)

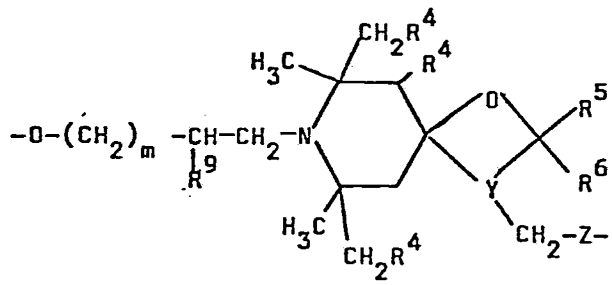

(Va)

dans laquelle $R^4$, $R^5$, $R^6$ et Y ont les sens indiqués ci-dessus, Z est une liaison ou représente —CH(OH)— et $R^9$ représente un groupe OH, et a les significations de $R^1$, ou bien A représente un groupe répondant aux formules (Vb) ou (Vc)

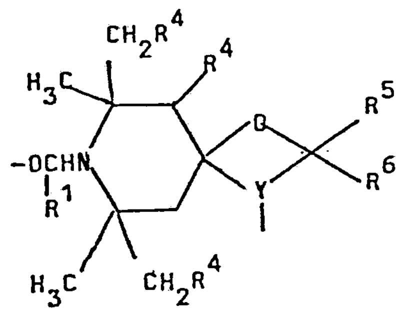

(Vb)

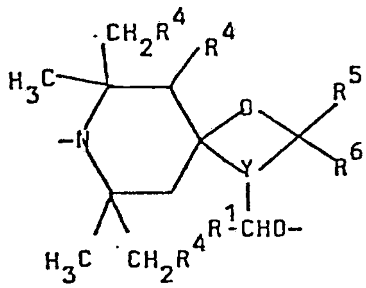

(Vc)

l'oxygène étant relié à $R^2$, et $R^1$, $R^4$, $R^5$, $R^6$ et Y ayant les sens indiqués ci-dessus, dans des produits oligomères ou polymères dans lesquels $n > 1$, $R^2$ représente comme groupe terminal de l'hydrogène, $R^3$ un radical di ou trifonctionnel, parmi les groupes indiqués pour $R^3$ et A est un groupe radical bifonctionnel pris parmi les groupes indiqués pour A, et représente un groupe de formule

$$R^2—A—CH(R^1)—O—$$

dans laquelle $R^1$, $R^2$ et A ont les sens indiqués ci-dessus, ou bien E n'a pas de signification et au moins l'un des radicaux $R^1$, $R^2$ ou A contient un système diazaspirodécane répondant aux formules (II) ou (V), ou bien E représente un groupe terminal qui peut être un groupe ester alkylique inférieur ayant la forme de l'ester méthylique ou éthylique, un groupe —NCO ou un halogène, procédé caractérisé en ce que :

a) on fait réagir un diazaspirodécane répondant aux formules générales (VIa) ou (VIb)

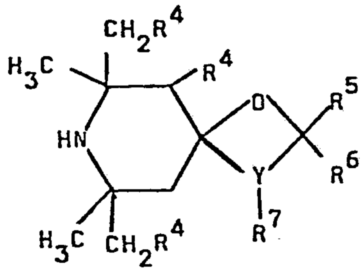

(VIa)

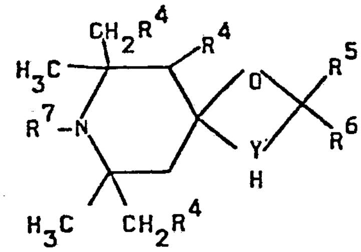

(VIb)

(dans lesquelles $R^4$, $R^5$, $R^6$, $R^7$ et Y ont les sens indiqués à la revendication 1) en présence d'un catalyseur à des températures élevées dans un solvant organique avec 1,0 à 1,5 fois la quantité équivalente d'un époxyde de formule (VIIa), ou bien 1,0 à 3 fois la quantité équivalente d'un aldéhyde de formule (VIIb) :

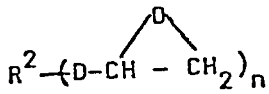

(VIIa)

$$R^2-(CHO)_n$$

(VIIb)

$R^2$ ayant dans la formule (VIIb) le sens indiqué à la revendication 1 et pouvant en outre dans la formule (VIIa), être un groupe répondant aux formules (II), n vaut 1, 2 ou 3 et D représente une liaison, ou bien un groupe méthylène ou $-OCH_2-$ dont —O— est relié à $R^2$, puis on fait réagir les alcools ainsi obtenus, si l'on souhaite, pour préparer des produits suivants ayant un poids molaire défini, également en présence d'un catalyseur et éventuellement d'un accepteur d'acide, à des températures élevées dans un solvant, avec l'équivalent mais, pour préparer des produits suivants oligomères ou polymères avec la quantité équimolaire d'un composé bifonctionnel de formule (VIII)

$$R^3-(T)_m \qquad (VIII)$$

dans laquelle $R^3$ a le sens indiqué à la revendication 1, m vaut 1, 2 ou 3 et T représente un halogène, un groupe ester alkylique inférieur, —COCl, —OH ou —NCO, ou

b) on fait réagir à 100 °C un composé de formule (VIIa), dans laquelle n vaut 1, D représente $-CH_2-$ et $R^2$ est un groupe de formule (IIa) ou (IIb), dans laquelle B représente une liaison, avec l'acide formique, qui doit être présent en une quantité molaire triple à décuple, et sert en même temps de solvant, on saponifie ensuite en milieu alcalin l'ester formé et, si on le souhaite, on soumet l'alcool ainsi obtenu, comme indiqué ci-dessus, avec addition d'un composé de formule (VIII) à une oligomérisation ou à une polymérisation.

2. Utilisation des composés, obtenus selon la revendication 1, de formule générale (I) pour stabiliser des polymères synthétiques.

3. Utilisation selon la revendication 2, caractérisée en ce que le polymère est une polyoléfine, un polyacrylate ou un polyméthacrylate, ou un homopolymère ou copolymère du styrène.

4. Utilisation selon la revendication 2, caractérisée en ce que le polymère constitue la partie solide d'un vernis ou d'une peinture.

5. Procédé de stabilisation de polymères synthétiques contre l'influence dégradante de la lumière, procédé caractérisé en ce qu'on ajoute au polymère, éventuellement en plus de substances connues jusqu'à présent, à rôle stabilisant, 0,01 à 5 parties en poids, par rapport au polymère, d'un stabilisant obtenu selon la revendication 1.

6. Polymères synthétiques stabilisés à l'encontre d'une décomposition par l'ultraviolet, et qui contiennent 0,01 à 5 parties en poids, par rapport au polymère, d'un stabilisant obtenu selon la revendication 1.